(19) Europäisches Patentamt / European Patent Office / Office européen des brevets

(11) **EP 4 481 054 A1**

(12) **EUROPEAN PATENT APPLICATION**

(43) Date of publication:
**25.12.2024 Bulletin 2024/52**

(21) Application number: **24176483.6**

(22) Date of filing: **17.05.2024**

(51) International Patent Classification (IPC):
**C12Q 1/04** (2006.01)

(52) Cooperative Patent Classification (CPC):
**C12Q 1/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.06.2023 SE 2350781**

(71) Applicants:
• **Sysmex Astrego AB**
  **756 51 Uppsala (SE)**
• **SYSMEX CORPORATION**
  **Kobe-shi**
  **Hyogo 651-0073 (JP)**

(72) Inventors:
• **Baltekin, Özden**
  **741 93 Knivsta (SE)**
• **Wistrand-Yuen, Erik**
  **754 74 Uppsala (SE)**
• **Knagge, Petter**
  **755 91 Uppsala (SE)**
• **Ranefall, Petter**
  **755 77 Vänge (SE)**

(74) Representative: **Barker Brettell Sweden AB**
  **Kungsbroplan 3**
  **112 27 Stockholm (SE)**

(54) **BACTERIA CLASSIFICATION AND ANALYSIS**

(57)  Bacterial cells from a biological sample are exposed to a test antimicrobial agent in addition to multiple other antimicrobial agents or mixtures. A MIC value of the test antimicrobial agent is determined based on a phenotypic characteristic response of the bacterial cells to the test antimicrobial agent. The bacterial cells are classified based on respective phenotype characteristic responses of the bacterial cells to the other antimicrobial agent or mixtures and the classification is used to determine a CBP value for the test antimicrobial agent. The MIC and CBP values are then determined and can be used to determine whether the bacterial cells from the biological sample are susceptible or not to the test antimicrobial agent.

START

S1 — EXPOSE BACTERIAL CELLS TO 1ST ANTIMICROBIAL AGENT/MIXTURE

S2 — EXPOSE BACTERIAL CELLS TO 2ND ANTIMICROBIAL AGENT/MIXTURE AND/OR TO SALT/SALT MIXTURE

S3 — EXPOSE BACTERIAL CELLS TO 3RD ANTIMICROBIAL AGENT/MIXTURE

S4 — DETERMINE MORPHOLOGY CHARACTERISTIC

S5 — CLASSIFY BACTERIAL CELLS

STOP

Fig. 2

## Description

TECHNICAL FILED

The present invention generally relates to bacteria classification and analysis.

BACKGROUND

Antibiotic susceptible testing (AST), also referred to as antibiotic sensitivity testing, is the measurement of the susceptibility of bacteria to antibiotics. Susceptibility testing results allows a clinician to select a suitable antibiotic or a mixture of antibiotics based on knowledge of the disease causing bacteria and their susceptibilities and resistances.

Initial AST methods were phenotypic methods involving exposing bacteria to antibiotics on agar plates or dilution in agar or broth. Such phenotypic methods include the disc diffusion method, also called the Kirby-Bauer method, in which bacteria are cultured on agar plate, and the bacterial growth near antibiotic-impregnated discs is observed. If the antibiotic inhibits microbial growth, a clear ring, or zone of inhibition, is seen around the disc. The bacteria are then classified as sensitive, intermediate, or resistant to an antibiotic by comparing the diameter of the zone of inhibition to defined thresholds, which correlate with minimum inhibitory concentrations (MICs). Other phenotypic methods include gradient methods, such as Etest, which use a plastic strip impregnated with different concentrations of antibiotics placed on agar and bacterial growth is viewed after a period of incubation. The MIC can be identified based on the intersection of the teardrop-shaped zone of inhibition with the marking on the strip.

A major disadvantage with the above exemplified phenotypic methods is that they require bacteria culturing, typically at least overnight, in order to get a visual response. Hence, it takes comparatively long period of time before the results of the AST is available. Another disadvantage is that generally only a single antibiotic or a low number of antibiotics can be tested in a single AST test.

Another group of AST methods are genetic methods based on polymerase chain reaction (PCR), deoxyribonucleic acid (DNA) microarrays, or DNA chips. These genetic methods do not look at the phenotypic response of bacteria to antibiotics but rather analyze whether the bacteria possess genes that confer antibiotic resistance. The genetic methods have advantage over culturing-based phenotypic methods in terms of being more rapid. Disadvantages include required knowledge of resistance genes to be tested, expensive and typically require specifically trained personnel. In addition, sometimes genotypic profile of detected resistance genes does not always match the resistance profile seen with phenotypic method.

Microfluidic devices and chips have been proposed to rapidly and in parallel monitor the phenotypic response of bacteria to a set of antibiotics. AST based on microfluidics is being rapid and does not suffer from the disadvantages associated with many genetic methods.

A prior art microfluidic device, denoted the "Mother Machine", is disclosed in Wang et al., Current Biology 2010, 20: 1099-1103. The Mother Machine allows for monitoring cells in many different cell channels in parallel.

Further microfluidic devices that are useful for analysis of biological samples are shown in U.S. Patent Nos. 10,041,104; 10,570,437 and 10,913,969.

Baltekin et al., PNAS 2017, 114(34): 9170-9175 discloses a fast antibiotic susceptible testing (AST) test, FASTest, using a microfluidic device.

U.S. Patent No. 8,828,680 discloses an automated assay methods and systems for the determination of antimicrobial susceptibilities and microorganism identification by using a hybrid system that combines turbimetric and fluorescence determinations. The test systems include a plurality of reaction chambers for performing the biochemical tests. Each reaction chamber is disposed to receive or contain one or more assay reagents, which comprise a substrate for at least one enzyme that is unique to a microorganism family, genus, and/or species. The substrate, if acted on by the enzyme, results in formation of a detectable product which is related to the identity of a microorganism in a sample.

There is still a need for an efficient classification and analysis of bacterial cells, such as useful in connection with antimicrobial agent susceptibility testing.

SUMMARY

It is a general objective to enable bacterial classification.

It is another objective to enable analyze bacterial cells to determine minimum inhibitory concentration and clinical breakpoint values.

These and other objectives are met by the embodiments disclosed herein.

The invention is defined in the independent claims. Further embodiments are defined in the dependent claims.

An aspect of the invention relates to a method for classifying bacteria. The method comprises exposing bacterial cells from a biological sample to a first antimicrobial agent or mixture and exposing bacterial cells from the biological sample to (i) a second antimicrobial agent or mixture and/or (ii) to a salt or salt mixture. The method further comprises classifying the bacterial cells from the biological sample at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture.

Another aspect of the invention relates to a method for analyzing bacteria. The method comprises exposing bacterial cells from a biological sample to a test antimicrobial agent. The method also comprises classifying the bacterial cells based on the method for classifying bacteria above. The method further comprises determining a clinical breakpoint (CBP) for the test antimicrobial agent based on a result of classifying the bacterial cells. The method additionally comprises determining a minimum inhibitory concentration (MIC) of the test antimicrobial agent based on a phenotype characteristic response of the bacterial cells to the test antimicrobial agent. The method also comprises comparing the MIC with the CBP.

A further aspect of the invention relates to an analyzer comprising a pressure source configured to apply pressure to a microfluidic device, a processor and a memory comprising instructions executable by the processor to cause the processor to control the pressure source to expose bacterial cells from a biological sample to a first antimicrobial agent or mixture in the microfluidic device and to control the pressure source to expose bacterial cells from the biological sample to (i) a second antimicrobial agent or mixture and/or (ii) to a salt or salt mixture in the microfluidic device. The processor is also caused to classify the bacterial cells from the biological sample at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture.

Yet another aspect of the invention relates to an analyzer comprising a pressure source configured to apply pressure to a microfluidic device a processor and a memory comprising instructions executable by the processor to cause the processor to control the pressure source to expose bacterial cells from a biological sample to a test antimicrobial agent in the microfluidic device, to control the pressure source to expose bacterial cells from the biological sample to a first antimicrobial agent or mixture in the microfluidic device and to control the pressure source to expose bacterial cells from the biological sample to (i) a second antimicrobial agent or mixture and/or (ii) to a salt or salt mixture in the microfluidic device. The processor is also caused to classify the bacterial cells at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture. The processor is further caused to determine a CBP for the test antimicrobial agent based on a result of classifying the bacterial cells and determine a MIC of the test antimicrobial agent based on a phenotype characteristic response of the bacterial cells to the test antimicrobial agent. The processor is additionally caused to compare the MIC with the CBP.

The present invention enables classification of bacterial cells from a biological sample and where such a classification can be used to determine a CBP for the bacterial cells and an antimicrobial agent. The present invention also enables determination of MIC of the antimicrobial agent together with determining CBP. The invention thereby enables verification whether the antimicrobial agent would be clinically effective in treating a bacterial infection by the bacterial cells in a patient.

BRIEF DESCRIPTION OF THE DRAWINGS

The embodiments, together with further objects and advantages thereof, may best be understood by making reference to the following description taken together with the accompanying drawings, in which:

Fig. 1 schematically illustrates MIC based classification of susceptible (S), resistant (R) and intermediate (I) phenotypes according to clinical breakpoints from epidemiological studies.

Fig. 2 is a flow chart illustrating a method for classifying bacteria according to an embodiment.

Fig. 3 is a flow chart illustrating a subroutine of the classifying step S5 in Fig. 2 according to an embodiment.

Fig. 4 is a flow chart illustrating a subroutine of the determining gram classification step S10 in Fig. 3 according to an embodiment.

Fig. 5 schematically illustrates a subroutine of the determining gram classification step S10 in Fig. 3 according to an embodiment.

Fig. 6 schematically illustrates a subroutine of the determining genus classification step S11 and the classifying step S12 in Fig. 3 according to an embodiment.

Fig. 7 is a flow chart illustrating a subroutine of the determining gram classification step S10 in Fig. 3 according to an embodiment.

Fig. 8 is a flow chart illustrating a subroutine of the determining genus classification step S11 in Fig. 3 according to an embodiment.

Fig. 9 is a flow chart illustrating a subroutine of the classifying step S12 in Fig. 3 according to an embodiment.

Figs. 10-13 schematically illustrate subroutines of the classifying step S6 in Fig. 1 according to various embodiments.

Fig. 14 is a flow chart illustrating a method for analyzing bacteria according to an embodiment.

Fig. 15 is a flow chart an additional optional step of the method shown in Fig. 14 according to an embodiment.

Fig. 16 schematically illustrates dose-response curves for bacterial cells exposed to various concentrations of antibiotics.

Fig. 17 schematically illustrates correlation between impact concentrations determined from the dose-response curves in Fig. 16 and reference MIC values.

Figs. 18A to 18C schematically illustrate attaching a microfluidic chip to a chip carrier (18A, 18B) and a bottom view of the microfluidic chip attached to the chip carrier (18C).

Fig. 19 schematically illustrates arrangement of the microfluidic chip and chip carrier in a substrate.

Fig. 20 schematically illustrates a microfluidic device.

Fig. 21 is a close-up view of a set of cell channels in the microfluidic device connected to a processor configured to control fluid flows of fluid circuitry of the microfluidic device.

Fig. 22 illustrates operational details of a microfluidic device with bacterial cells flowing into the cell channels during capture.

Fig. 23 is a phase contrast microscopy image of bacterial cells captured into cell channels (darker segments represent cells, lighter segments represent empty part of cell channels) with 100× magnification.

Fig. 24 schematically illustrates an analyzer configured to perform the methods according to the embodiments.

Fig. 25 is a block diagram illustrating an embodiment of the analyzer.

Fig. 26 illustrates (26A) gram classification of biological samples (N=676) into gram positive or gram negative and (26B, 26C) genus classification of biological samples (N=130).

DETAILED DESCRIPTION

Throughout the drawings, the same reference numbers are used for similar or corresponding elements.

The present invention generally relates to bacteria classification and analysis.

The traditional approach of determining whether an antimicrobial agent, such as an antibiotic, is effective in treating an infection by a particular bacterial strain is to compare the minimum inhibitory concentration (MIC) of the antimicrobial agent as determined for the particular bacterial strain with the clinical breakpoint (CBP) of the antimicrobial agent as determined for a bacterial grouping encompassing the particular bacterial strain. Briefly, if the MIC is below the CBP, the bacterial strain is classified as susceptible to the antimicrobial agent, whereas if the MIC is above the CBP, the bacterial strain is classified as resistant to the antimicrobial agent.

MIC is the lowest concentration of an antimicrobial agent that prevents visible growth of a bacterial strain or isolate. MIC values are traditionally determined by antibiotic susceptible testing (AST), such as broth dilution assays or the Etest. A MIC value as determined by AST, however, does notperse define whether an infection caused by the bacterial strain or isolate is likely to be treatable in a patient by the antimicrobial agent. Clinically, the more relevant value for treatment is the so-called CBP. CBP is the concentration of an antimicrobial agent used to define whether an infection by a particular bacterial strain or isolate is likely to be treatable in a patient. Typically, these are defined as susceptible or resistant to an antimicrobial agent. CBP concentrations are different from antimicrobial susceptibility tests, since they take into account what happens to an antimicrobial agent within the body, pharmacological and pharmacokinetic considerations, which will affect the clinical efficacy of an antimicrobial agent.

CBPs are defined and listed for orders, genera or species of bacteria. Hence, in order to determine whether an infection caused by a particular bacterial strain or isolate is treatable with an antimicrobial agent, not only the MIC of the antimicrobial agent for the bacterial strain or isolate needs to be determined but also the order, genus or species of the bacterial strain or isolate. Information of order, genus or species is needed in order to select the correct CBP to be compared to the determined MIC to verify whether the particular bacterial strain or isolate is classified as susceptible or resistant. Hence, in the prior art, two different analyses are needed, AST for determination of MIC and another analysis for order, genus or strain identification, such as using morphological tests, biochemical tests, serotyping, and/or genetic tests.

The present invention enables, in a single analysis, typically in a microfluidic device, determination of both MIC value of a test antimicrobial agent and identification or classification of the bacterial strain or isolate as needed for determining CBP. Accordingly, a single device and analysis could be used to provide the information required in order to determine whether an infection in a patient caused by the particular bacterial strain or isolate is likely to be treatable (susceptible) or not (resistant) using a test antimicrobial agent.

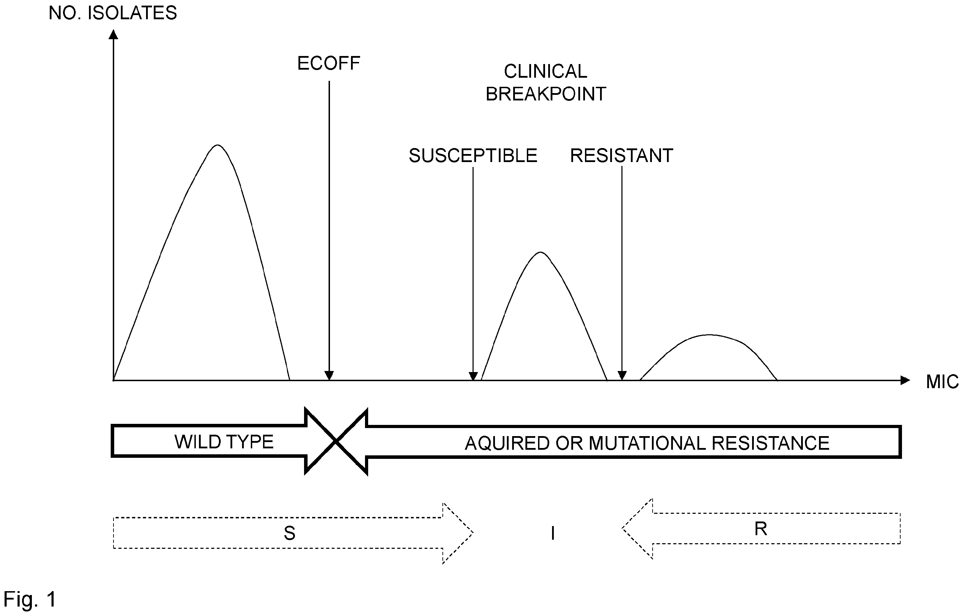

Generally, CBPs are defined as order/genus/-species-related antimicrobial concentration values that help in phenotypic susceptible/resistant (S/R) profile interpretation based on MIC values. Therefore, a bacterial strain or isolate with a MIC value lower than or equal to a susceptibility CBP value is considered susceptible (S), otherwise resistant (R). In some cases, a resistance CBP is also defined to distinguish for intermediate (I) phenotype, see Fig. 1. CBPs are made available and updated annually by specialized institutes, such as European Committee on Antimicrobial Susceptibility Testing (EUCAST) in Europe and Clinical Laboratory Standards Institute (CLSI) in the U.S.

Another key parameter value is represented by the epidemiological cut-off value (ECOFF), which is the highest MIC value for the screened bacteria without any resistance mechanism, i.e., wild type (WT) bacteria. ECOFF is very useful for the assessment of intrinsic resistance to particular antibiotics seen in some species and in establishing the CBPs.

Fig. 1 illustrates an example of MIC distribution and ECOFF value determination from EUCAST epidemiological study comprising several thousand isolates or strains of a particular bacterial species exposed to an antimicrobial agent.

"Biological sample" as used herein include any sample, preferably fluid sample, and more preferably liquid sample, comprising bacterial cells. The biological sample is preferably a body fluid sample, such as a body fluid sample taken from a patient, including a processed body fluid sample taken from a patient. Non-limiting, but illustrative, examples of such body fluid samples include urine, blood, plasma, serum, amniotic fluid, cerebrospinal fluid, lymph, saliva and synovial fluid. Alternatively, the biological sample could be a biological sample obtained from a solid body sample, such as a biopsy, in which bacterial cells of the solid body sample have been suspended or dispersed in a fluid, preferably a liquid, such as a culture medium.

"Antimicrobial agent" as used herein relates to any molecule, compound, composition or other agent that kills bacterial cells or stops, or at least reduces, growth of bacterial cells. Typical, but non-limiting, examples of such antimicrobial agents include drugs or medicaments, including drug candidates, such as antibiotics or other antibacterials. Illustrative, but non-limiting, examples of such antibiotics include penicillins, cephalosporins, polymyxins, rifamycins, lipiarmycins, sulfonamides, lincosamides, beta-lactams, macrolides, quinolones, tetracyclines and aminoglycosides.

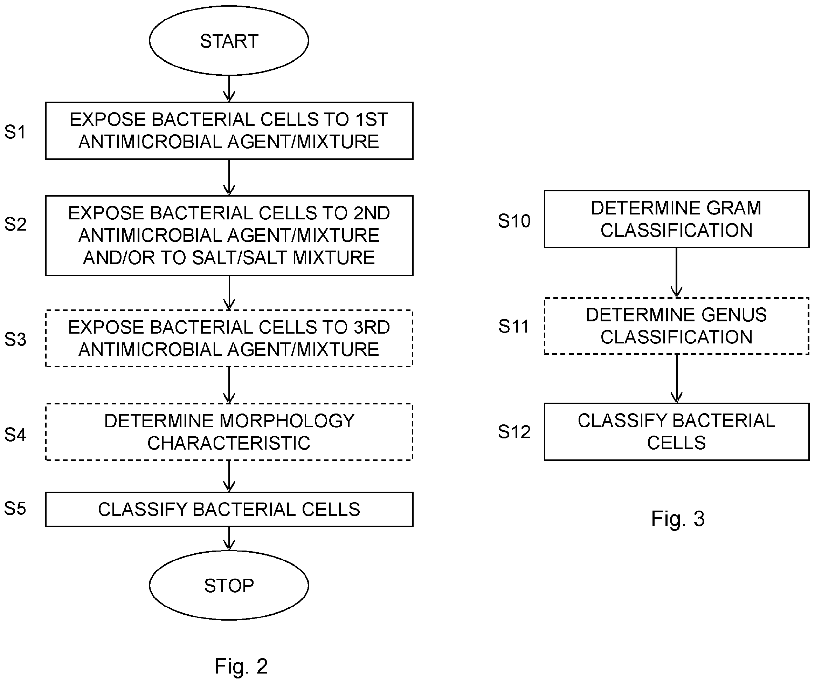

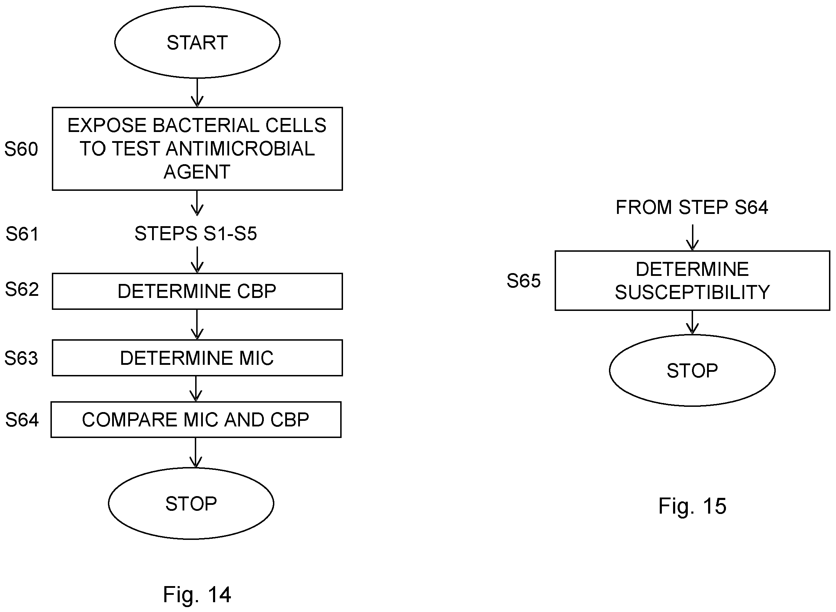

Fig. 2 is a flow chart illustrating a method for bacteria classification. The bacteria classification can be performed as a separate method or process in order to classify bacterial cells from a biological sample, such as to determine an order or genus of the bacterial cells. The classification of bacterial cells is advantageously used as a basis for determining or selecting CBP for the bacterial cells. In such a case, the bacteria classification can be a part of an analysis process or method, which is further disclosed herein with reference to Fig. 14.

The method for classifying bacteria in Fig. 2 comprises exposing bacterial cells from a biological sample to a first antimicrobial agent or mixture in step S1. Step S2 correspondingly comprises exposing bacterial cells from the biological sample to (i) a second antimicrobial agent or mixture and/or (ii) to a salt or salt mixture. The bacterial cells from the biological sample are then classified in step S5 at least based on (i) a phenotypic characteristic response of the bacterial cells to the first antimicrobial agent or mixture, and (iia) a phenotypic characteristic response of the bacterial cells to the second antimicrobial agent or mixture and/or (iib) a phenotypic characteristic response of the bacterial cells to the salt or salt mixture.

Steps S1 and S2 can be performed serially in any order but are preferably performed at least partly in parallel. In this latter preferred case, bacterial cells from the biological sample are exposed to the first antimicrobial agent or mixture in parallel with exposing other bacterial cells from the biological sample to the second antimicrobial agent or mixture and/or exposing other bacterial cells from the biological sample to the salt or salt mixture.

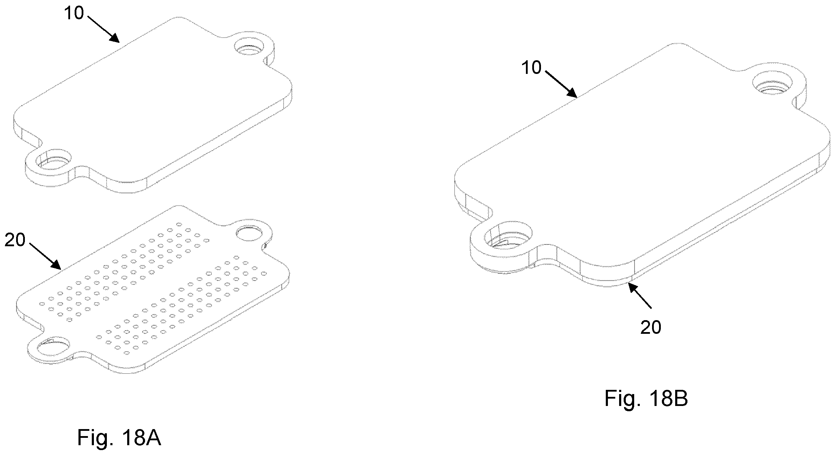

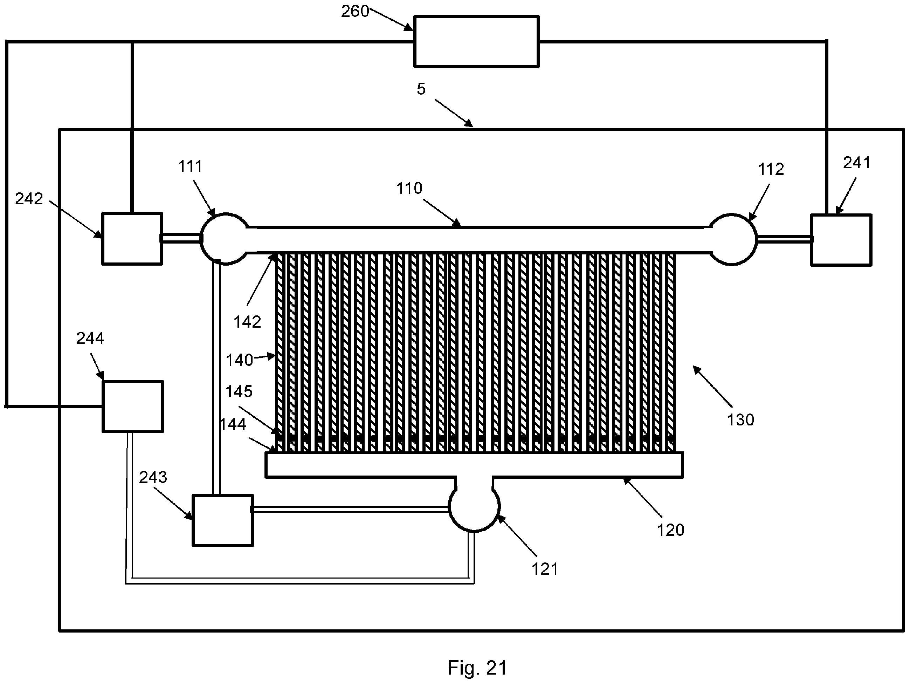

As an example of such parallel exposure to multiple, i.e., at least two, antimicrobial agents or mixtures is to load a biological sample comprising bacterial cells into cell channels 140 of a microfluidic chip 10, see Figs. 18A-18C, 21. The microfluidic chip 10 comprises a plurality of sets 130 of cell channels 140, a plurality of input channels 110 and a plurality of output channels 120. As is more clearly shown in Fig. 21, each input channel 110 is in fluid communication with a respective output channel 120 through the cell channels 140 of a respective set 130 of cell channels 140. The cell channels 140 comprise a respective channel restriction 145 configured to restrict bacterial cells entering the cell channels 140 from reaching the output channel 120.

In such a case, step S1 could comprise exposing bacterial cells captured in cell channels 140 of a first set or sets 130 to the first antimicrobial agent or mixture and step S2 comprises exposing bacterial cells captured in cell channels 140 of a second set or sets 130 to the second antimicrobial agent or mixture and/or exposing bacterial cells captured in cell channels 140 of a fourth set or sets 130 to the salt or salt mixture. The bacterial cells captured in and thereby present in the cell channels 140 of the first set or sets 130 are exposed to the first antimicrobial agent or mixture preferably simultaneously as the bacterial cells captured in and thereby present in the cell channels 140 of the second set or sets 130 are exposed to the second antimicrobial agent or mixture and/or the bacterial cells captured in the cell channels 140 of the fourth set or sets 130 are exposed to the salt or salt mixture.

A single respective antimicrobial agent could be used in steps S1 and S2. Alternatively, a mixture of two or more different antimicrobial agents could be used in any of these two steps S1 and S2.

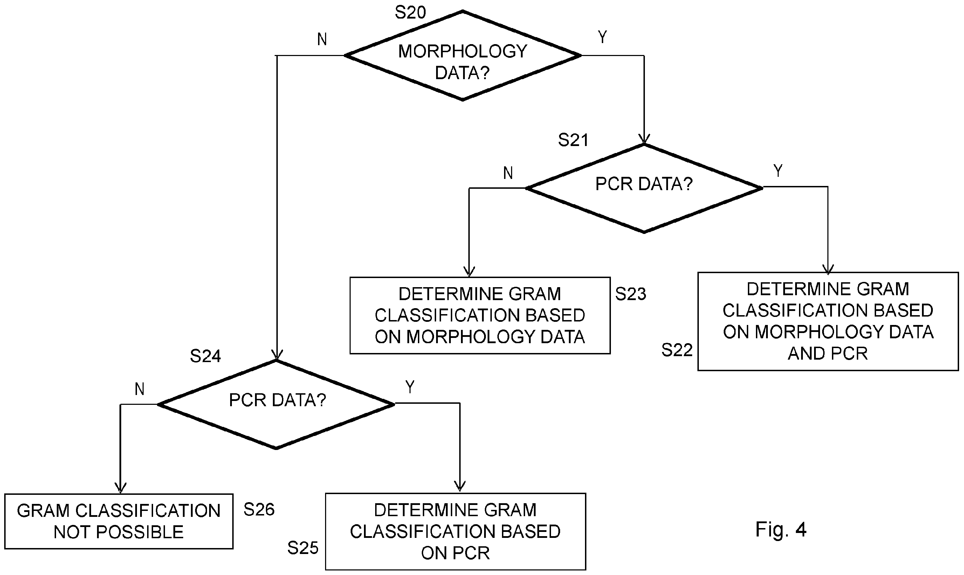

Fig. 3 is a flow chart illustrating a subroutine of the classifying step S5 in Fig. 2. The method continues from any of the steps S2 to S4 in Fig. 2. A next step S10 comprises determining a gram classification for the bacterial cells at least based on the phenotype characteristic response of the bacterial cells to an antimicrobial agent or mixture, at least one morphology characteristic of the bacterial cells from the biological sample, or the phenotype characteristic response of the bacterial cells to the salt or salt mixture. Hence, a gram classification is, in this embodiment, determined for the bacterial cells based on at least one of the phenotype characteristic response of the bacterial cells to the antimicrobial agent or mixture, the morphology characteristic of the bacterial cells from the biological sample, and the phenotype characteristic response of the bacterial cells to the salt or salt mixture. This is further described herein with reference to Fig. 4.

The phenotype characteristic response of the bacterial cells to the antimicrobial agent or mixture as used in step S10 could the phenotype characteristic response of the bacterial cells to the first antimicrobial agent or mixture, to which the bacterial cells are exposed in step S1 or indeed to another antimicrobial agent or mixture, such as the second antimicrobial agent or a third antimicrobial agent, to which the bacterial cells may be exposed to in steps S2 and S3 of Fig. 2, respectively. In a preferred embodiment, step S10 comprises determining the gram classification for the bacterial cells at least based on the phenotype characteristic response of the bacterial cells to the first antimicrobial agent or mixture, at least one morphology characteristic of the bacterial cells from the biological sample, or the phenotype characteristic response of the bacterial cells to the salt or salt mixture.

In an embodiment, the method also comprises optional step S11. This step S11 comprises determining a genus classification of the bacterial cells based on the phenotype characteristic response of the bacterial cells to the second antimicrobial agent or mixture and/or the phenotype characteristic response of the bacterial cells to the salt or salt mixture, and preferably also based on the phenotype characteristic response of the bacterial cells to the first antimicrobial agent or mixture.

These steps S10 and S11 can be performed serially in any order or at least partly in parallel.

The present embodiment then also comprises classifying the bacterial cells in step S12 based on the gram classification determined in step S10 and the optional genus classification determined in step S11.

Hence, in this embodiment, the (first) antimicrobial agent or mixture, the at least one morphology and/or the salt or salt mixture is used to determine a gram classification for the bacterial cells, i.e., determine whether the bacterial cells are likely to be gram positive or gram negative.

In bacteriology, gram-positive bacteria are bacteria that give a positive result in a Gram stain test, which is traditionally used to quickly classify bacteria into two broad categories according to their type of cell wall. Gram-positive bacteria take up the crystal violet stain used in the Gram stain test, and then appear to be purple-colored when seen through an optical microscope. This is because the thick peptidoglycan layer in the bacterial cell wall retains the crystal violet stain after it is washed away from the rest of the sample, in the decolorization stage of the Gram stain test. Conversely, gram-negative bacteria cannot retain the crystal violet stain after the decolorization step. Alcohol used in this stage degrades the outer membrane of gram-negative cells, making the cell wall more porous and incapable of retaining the crystal violet stain. The peptidoglycan layer of gram-negative cells is much thinner and sandwiched between an inner cell membrane and a bacterial outer membrane, causing them to take up the counterstain (safranin or fuchsine) and appear red or pink.

The antimicrobial agent or mixture to be used in step S10, preferably the first antimicrobial agent, is preferably selected to be an antimicrobial agent or mixture that affects gram-positive bacteria but not significantly affecting gram-negative bacteria or affects gram-negative bacteria but not significantly affecting gram-positive bacteria. In the former case, the (first) antimicrobial agent or mixture is a gram-positive antimicrobial agent that is effective against gram-positive bacteria but not effective, or at least not equally effective, against gram-negative bacteria. Illustrative, but non-limiting, examples of gram-positive antimicrobial agents include glycopeptides, vancomycin and teicoplanin. In the latter case, the (first) antimicrobial agent or mixture is a gram-negative antimicrobial agent that is effective against gram-negative bacteria but not effective, or at least not equally effective, against gram-positive bacteria. Illustrative, but non-limiting, examples of gram-negative antimicrobial agents include cephalosporins, such as cefepime, β-lactamase inhibitor penicillins, such as piperacillin, tazobactam, ticarcillin, clavulanate, and carbapenems, such as imipenem, cilastatin, meropenem, ertapenem. An illustrative example of the (first) antimicrobial agent or mixture is a combination or mixture of linezolid (LIN) and vancomycin (VAN).

The second antimicrobial agent or mixture is used, preferably together with the first antimicrobial agent or mixture, to determine an optional genus classification for the bacterial cells in step S11, i.e., determine a genus to which the bacterial cells from the biological sample belongs.

Genus is a taxonomic rank used in the biological classification of organisms and viruses. In the hierarchy of biological classification, genus comes above species and below family.

The second antimicrobial agent or mixture is therefore selected to be effective against bacteria of a first genus but not effective, or not equally effective, against bacteria of a second genus. Illustrative, but non-limiting, examples of such antimicrobial agents include amoxicillin and clavulanic acid (AMC) and fosfomycin (FOS), which are generally effective against *Staphylococcus* but less effective against *Enterococcus.*

The classification of the bacterial cells is then done in step S12 based on the gram classification and optionally based on the genus classifications as determined in steps S10 and S11.

In an embodiment, step S10 in Fig. 3 comprises determining the gram classification for the bacterial cells based on the phenotype characteristic response of the bacterial cells to the (first) antimicrobial agent or mixture and at least one morphology characteristic of bacterial cells from the biological sample, also referred to as morphology classification herein.

Hence, in this embodiment, not only the phenotype characteristic response of the bacterial cells to the (first) antimicrobial agent or mixture but also the morphology of the bacterial cells as presented by the at least one morphology characteristic is used to determine the gram classification in step S10.

Morphology characteristic as used herein refers to the form, shape, size and/or structure of the bacterial cells. The at least one morphology characteristic could thereby represent the form or shape of the bacterial cells, the size of the bacterial cells or the structural of the bacterial cells. As an example, the at least one morphology characteristic could be rod or rod-shaped (cylindrical shape) versus cocci or cocci-shaped (spherical shape). The at least one morphology characteristic is preferably determined based on at least one image of the bacterial cells. In such an embodiment, the at least one morphology characteristic could be determined based on at least one image of bacterial cells captured in cell channels 140 of a reference or control set or sets 130 in the microfluidic chip 10. The bacterial cells captured in the reference set or sets 130 are preferably not exposed to any antimicrobial agent or mixture. For instance, the bacterial cells captured in cell channels 140 of the reference set or sets 130 could be exposed to a culture medium in parallel with exposing bacterial cells captured in cell channels 140 of the first set or sets 130 to the first antimicrobial agent or mixture and exposing bacterial cells captured in cell channels 140 of the second set or sets 130 to the second antimicrobial agent or mixture and/or exposing bacterial cells captured in cell channels 140 of the fourth set or sets 130 to the salt or salt mixture.

Fig. 4 is a flow chart illustrating a subroutine of step S10 in Fig. 3 according to an embodiment. In this embodiment, an optional validation of the data is first made in steps S20, S21 and S24. Step S20, thus, comprises determining whether valid data of the at least one morphology characteristic of bacterial cells from the biological sample is available. If such valid data is available, the method continues to step S21. Step S21 comprises determining whether valid data of the phenotype characteristic response (PCR) of the bacterial cells to the

(first) antimicrobial agent or mixture is available. If valid data is not available in step S20, the method continues to step S24. This step S24 is performed in the same way as step S21. In another embodiment of the subroutine, an initial check is first made to determine whether valid data of the phenotype characteristic response of the bacterial cells to the (first) antimicrobial agent or mixture is available and then a following check is made to determine whether valid data of the at least one morphology characteristic of bacterial cells from the biological sample is available. In such an embodiment, the order of step S20 and steps S21 and S24 are thereby changed.

In the subroutine shown in Fig. 4, the determination of the gram classification is performed in accordance with any of steps S22, S23, S25 and S26 depending on which valid data is available. In more detail, the determination of the gram classification is performed as shown in step S22 if valid data of the at least one morphology characteristic of the bacterial cells and valid data of the phenotype characteristic response of the bacterial cells to the (first) antimicrobial agent or mixture are available. Step S22 comprises determining the gram classification for the bacterial cells at least based on the phenotype characteristic response of the bacterial cells to the (first) antimicrobial agent or mixture and the at least one morphology characteristic of the bacterial cells from the biological sample.

However, if valid data of the at least one morphology characteristic of the bacterial cells is available but no valid data of the phenotype characteristic response of the bacterial cells to the (first) antimicrobial agent or mixture is available, the determination of the gram classification is performed as shown in step S23. This step S23 comprises determining the gram classification for the bacterial cells at least based on the at least one morphology characteristic of the bacterial cells from the biological sample.

The determination of the gram classification is performed in accordance with step S25 if valid data of the phenotype characteristic response of the bacterial cells to the (first) antimicrobial agent or mixture is available but no valid data of the at least one morphology characteristic of the bacterial cells is available. Step S25 comprises determining the gram classification for the bacterial cells at least based on the phenotype characteristic response of the bacterial cells to the (first) antimicrobial agent or mixture.

If no valid data is available as determined in steps S20 and S24, then no gram classification is possible as concluded in step S26.

No valid data for the phenotype characteristic response or the at least one morphology characteristic could be due to various reasons. For instance, air bubbles or non-cell material could be present in the cell channels 140 of the microfluidic chip 10. Such air bubbles or non-cell material could interfere with determining the morphology characteristic(s) of the bacterial cells and/or the phenotype characteristic response of the bacterial cells to the (first) antimicrobial agent or mixture. Another reason for no valid data could be that too few bacterial cells are captured in the cell channels 140 of the control set or sets 130 or of the first set or sets 130 in the microfluidic chip 10. Yet another reason could be that the at least one morphology characteristic or the phenotype characteristic response of the bacterial cells to the first antimicrobial agent or mixture is noisy for other reasons.

In a particular embodiment, step S2 in Fig. 2 comprises exposing bacterial cells from the biological sample to a salt or salt mixture and preferably also exposing bacterial cells from the biological sample to the second antimicrobial agent or mixture.

In this embodiment, steps S1, S2 can be performed serially in any order but are preferably performed at least partly in parallel. In this latter preferred case, bacterial cells from the biological sample are exposed to the first antimicrobial agent or mixture in parallel with exposing other bacterial cells from the biological sample to the second antimicrobial agent or mixture and other bacterial cells from the biological sample to the salt or salt mixture.

In this embodiment, step S10 in Fig. 3 comprises determining the gram classification for the bacterial cells based on a phenotype characteristic response of the bacterial cells to the salt or salt mixture if no valid data of the at least one morphology characteristic of the bacterial cells and no valid data of the phenotype characteristic response of the bacterial cells to the (first) antimicrobial agent or mixture are available.

In the above-described embodiments, various ways of determining the gram classification can be used depending on what valid data that is available from the analysis. Thus, in a preferred case when all necessary valid data is available, the determination of the gram classification is done in accordance with step S22, whereas if merely one of the morphology characteristic(s) and the phenotype characteristic response of the bacterial cells to the (first) antimicrobial agent or mixture is available then the determination of the gram classification is done in accordance with step S23 or S25. The usage of the phenotype characteristic response of the bacterial cells to the salt or salt mixture could then be seen as a fallback alternative if no other valid data is available when determining the gram classification. Hence, in such an embodiment, step S26 as shown in Fig. 4 is replaced by determining gram classification at least based on the phenotype characteristic response of the bacterial cells to the salt or salt mixture.

In the above-described embodiments using the at least one morphology characteristic of the bacterial cells, the method as shown in Fig. 2 preferably comprises an additional step S4 comprising determining the at least one morphology characteristic.

In this embodiment, steps S1, S2 and S4 can be performed serially in any order but are preferably performed at least partly in parallel. In this latter preferred case, bacterial cells from the biological sample are exposed to the first antimicrobial agent or mixture in parallel with exposing other bacterial cells from the biological sample to the second antimicrobial agent or mixture and/or other bacterial cells from the biological sample to the salt or salt mixture in parallel with taking at least one image of bacterial cells that are preferably not exposed to any antimicrobial agent or mixture or to the salt or salt mixture.

The at least one morphology characteristic is preferably determined in step S4 based on at least one image of bacterial cells from the biological sample. Step S4 is preferably performed using a neural network on images of cell channels 140 comprising captured bacterial cells.

In a particular embodiment, step S4 comprises monitoring bacterial cells from the biological sample at one or preferably multiple time instances while the bacterial cells are not exposed to any antimicrobial agent or mixture. In clear contrast, the bacterial cells are instead preferably exposed to a solvent, preferably a culture medium. The at least one morphology characteristic is then determined in step S4 based on monitoring the bacterial cells at the multiple time instances.

In a preferred embodiment, monitoring of the bacterial cells is performed by monitoring bacterial cells from the biological sample captured in cell channels 140 of a reference set or sets 130 in a microfluidic device 5 or a microfluidic chip 10. In such a case, the at least one morphology characteristic is determined by, for each cell channel 140 of the multiple cell channels 140, determining, at each time instance of the multiple time instances, at least one tentative channel-specific morphology characteristic, determining at least one channel-specific morphology characteristic of the bacterial cells captured in the cell channel 140 based on the tentative channel-specific morphology characteristics and determining the at least one morphology characteristic of the bacterial cells based on the channel-specific morphology characteristics.

In a particular embodiment, the at least one tentative channel-specific morphology characteristic is determined by determining, at each time instance of the multiple time instances, the at least one tentative channel-specific morphology characteristic based on an image of the bacterial cells in the cell channel 140 and a computer-implemented morphology classification model trained for classifying bacterial cells based on images of the bacterial cells.

In a particular embodiment, the at least one channel-specific morphology characteristic is then determined as a first channel-specific morphology classification, such as rod or rod-shaped, if at least a minimum percentage of the tentative channel-specific morphology characteristics indicates the first classification, as a second channel-specific morphology characteristics, such as cocci or cocci-shaped, if at least a minimum percentage of the tentative channel-specific morphology characteristics indicates the second classification, and optionally otherwise as indefinite. The minimum percentage is preferably a preselected value larger than 50 %, such as about 75 %. The at least one morphology characteristic of the bacterial cells is then preferably determined based on a quotient between the number of cell channels 140 classified as the first classification and the number of cell channels classified as the second classification.

Hence, in an embodiment, the morphology classification is based on a neural network (computer-implemented morphology classification model) trained for estimating morphology characteristic of bacterial cells based on images of individual cell channels 140 comprising bacterial cells with known bacterial morphology. In more detail, the neural network has been trained based on 421 datasets containing 96 datasets of captured cocci-shaped bacteria and 325 datasets of rod-shaped bacteria. From these datasets, all images of all cell channels 140 where the total length of the captured bacteria corresponded to 24 or more pixels and the growth rate was $\geq 0.005$ minutes$^{-1}$. This resulted in 9727960 cell channel images in total, of which 676106 images contained cocci-shaped bacteria and 9051854 images contained rod-shaped bacteria. The data was trimmed to make the datasets balanced, and to select -60% for training, and -20% for each of validation and test. When doing the splitting to train/validate/test, all cell channel images from the same initial dataset ended up in the same set train/validate/test in order to make these sets completely independent. A deep learning model was designed and trained using the train data. During each epoch (=iteration) of the training, the deep learning model was validated against the validate dataset and the weights of the deep learning model were frozen at the epoch that gave the best accuracy on the validate dataset. The deep learning model was tested on the independent test dataset and the resulting binary accuracy was 94.2%. This neural network generated a morphology classification of each individual cell channel 140 at each timepoint during the assay. For each cell channel 140, the classifications at every timepoint are summarized, and if the classification is consistent over time the cell channel 140 will be set to this morphology. If the morphology is inconsistent and switches between rod and cocci classification, it is set to "ambivalent" and excluded from the final morphology scoring. The cell channel classification is then summarized over all cell channels 140 in the reference set or sets 130 and a final morphology score is calculated based on the ratio between cell channels 140 with rods and cocci. For instance, if X cell channels 140 in the reference set or sets 130 are classified as rods and Y cell channels 140 in the reference set or sets 130 are classified as cocci then the ratio could be X/(X+Y) or Y/(X+Y). If the ratio is X/(X+Y) is above a defined threshold value, such as 0.75, the final morphology classification is, in this example, rods. Correspondingly, if the ratio Y/(X+Y) is above the defined threshold value, the final morphology classification is, in this example, cocci.

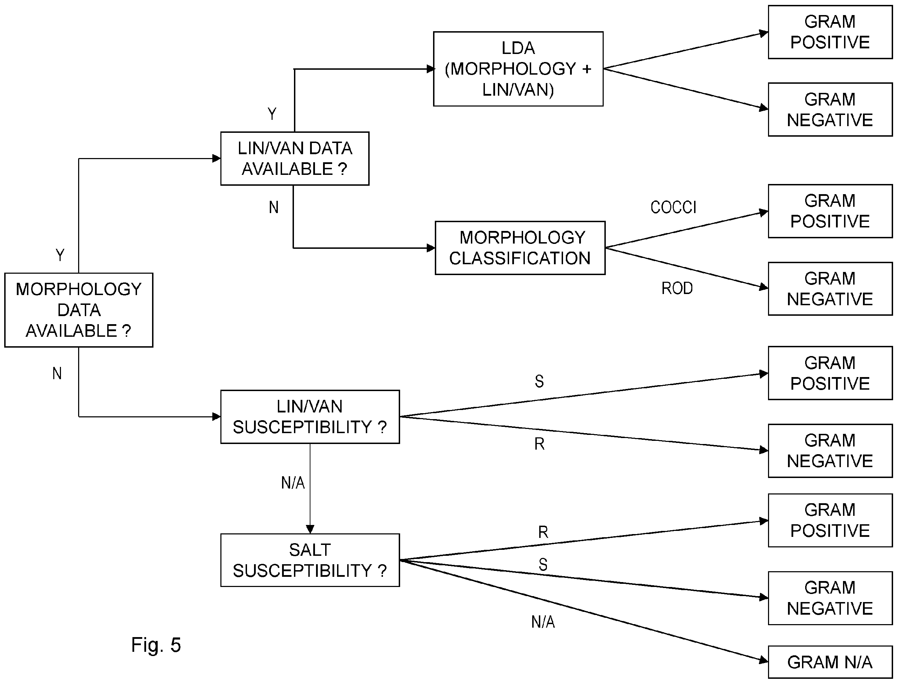

Fig. 5 schematically illustrate a subroutine of the step S10 in Fig. 3 according to an embodiment to determine a gram classification for any bacterial cells present in the sample. This embodiment is in particular adapted for classifying bacterial cells present in a biological sample, such as urine sample, taken from a subject suffering from UTI.

In this embodiment, bacterial cells from a biological sample are exposed to four different treatments corresponding to steps S1 to S3 in Fig. 2. The first treatment comprises a combination of linezolid (LIN) and vancomycin (VAN), the second treatment comprises amoxicillin and clavulanic acid (AMC), the third treatment comprises fosfomycin (FOS) and the fourth treatment comprises a salt treatment with sodium chloride (NaCl). The combination of LIN/VAN in the first treatment is generally effective against gram-positive bacteria but has no or little antibacterial activity against gram-negative bacteria. Correspondingly, salt treatment using NaCl is generally effective against gram-negative bacteria, but has less antibacterial activity against gram-positive bacteria, in particular little or no impact against *Staphylococcus*. AMC used in the second treatment has broad antibacterial activity against both gram-positive and gram-negative bacteria. It, though, generally has slower antibacterial activity against *Staphylococcus* as compared to against *Enterococcus*. FOS used in the third treatment has a broad antibacterial activity against both gram-positive and gram-negative bacteria. It, though, generally has a slower antibacterial activity against *Staphylococcus* as compared to against *Enterococcus*. Bacterial cells from the biological sample are preferably exposed to respective treatment in parallel. In this example, a respective impact parameter is determined for each treatment after a respective treatment period and then compared to a respective cutoff or threshold value. The respective treatment periods could be the same but are typically different and adapted to the particular antimicrobial agent or mixture or salt or salt mixture.

In an embodiment, the impact parameters are a respective reduction of normalized growth rate fitted to a logistic function for each antimicrobial agent or mixture (LIN/VAN, AMC, FOS) or a growth rate reduction in the salt treatment (not fitted data, but average normalized growth rate in a set time interval). In an embodiment, LINNAN is a single treatment, but for AMC and FOS the impact parameter is based on a "dose-response curve", a logistic curve fitted to the antibiotic impact at a set timepoint of all valid treatments of each antibiotic. The impact parameter is then defined as the reduction in normalized growth rate at a defined concentration based on the fitted curve.

The gram classification first verifies whether valid morphology data and valid LINNAN data are available, see steps S20 and S21 in Fig. 4. If valid morphology characteristic and LIN/VAN data are available, the gram classification is determined based on a linear discriminant analysis (LDA) of the morphology characteristic and the phenotypic characteristic response of the bacterial cells to LINNAN.

Valid morphology data means that at least a minimum number of cell channels 140 in the reference or control set or sets 130 in the microfluidic chip 10 have been classified as containing either, for instance, cocci or rods. This minimum number could represent a number of cell channels 140, such as 25, a minimum percentage of the cell channel 140 in the reference or control set or sets 130 as classified as containing, for instance, cocci or rods.

Reasons for the LIN/VAN data, or indeed data for any other antimicrobial agent or mixture treatment, to be invalid could be that the image analysis of the bacterial cells in the cell channel 140 detects air bubbles, that too few channels contain bacterial cells, that the number of captured cells deviate too much from the other treatments in the microfluidic chip 10 (indicating issues with that treatment), or that the growth rate data in that treatment is too noisy and that a logistic curve cannot be fitted to the data points without large errors.

LDA, also referred to as normal discriminant analysis (NDA) or discriminant function analysis, is a method used to find a linear combination of features that characterizes or separates two or more classes of objects or events. The resulting combination is used as a linear classifier to classify the bacterial cells as gram positive or gram negative.

In case no valid LIN/VAN data is available but valid morphology data is available, the gram classification is done based on the morphology characteristic of the bacterial cells, classifying the bacterial cells as gram positive or gram negative. For instance, if the morphology characteristic determined for the bacterial cells is rod or rod-shaped, then the gram classification is gram negative. Correspondingly, if the morphology characteristic determined for the bacterial cells is cocci or cocci-shaped, then the gram classification is gram positive.

Correspondingly, if no valid morphology data is available but valid LINNAN data is available, the classification of the bacterial cells as gram positive or gram negative is performed based on the impact parameter determined for the LINNAN combination. Hence, if the bacterial cells are determined to be susceptible to LINNAN then they are classified as gram positive, whereas if the bacterial cells are determined to be resistant against LINNAN then the bacterial cells are classified as gram negative.

The salt treatment as performed in step S2 of Fig. 2 is used as a fallback or auxiliary classification if no valid morphology or LIN/VAN data are available. In such a case, the bacterial cells are classified as gram negative if they are susceptible to the salt treatment and as gram positive if they are resistant against the salt treatment. In the event that no valid salt treatment data is available as well no gram classification can be made (not applicable (N/A)).

In another embodiment, step S10 in Fig. 3 preferably comprises determining the gram classification for the bacterial cells based on the phenotype characteristic response of the bacterial cells to the first antimicrobial agent or mixture and a phenotype characteristic response of the bacterial cells to the salt or salt mixture.

Hence, in this particular embodiment, the phenotype characteristic responses of the bacterial cells to the first antimicrobial agent or mixture and the salt or salt mixture are used to determine the gram classification in step S10, such as to determine whether the bacterial cells are gram positive or gram negative.

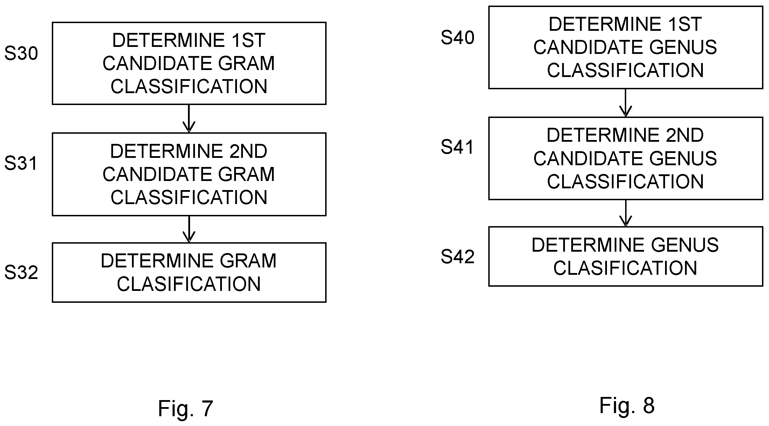

Fig. 7 is a flow chart illustrating a subroutine of step S10 in Fig. 3 according to an embodiment. A next step S30 comprises determining a first candidate gram classification for the bacterial cells based on the phenotype characteristic response of the bacterial cells to the first antimicrobial agent or mixture. Correspondingly, step S31 comprises determining a second candidate gram classification for the bacterial cells based on the phenotype characteristic response of the bacterial cells to the salt or salt mixture.

These steps S30 and S31 can be performed serially in any order or at least partly in parallel.

A next step S32 comprises, in an embodiment, determining the gram classification for the bacterial cells as a gram classification indicated by the first candidate gram classification and the second candidate gram classification if both the first candidate gram classification and the second candidate gram classification indicate the same gram classification.

This embodiment thereby determines a respective candidate gram classification for the bacterial cells based on the phenotype characteristic responses of the bacterial cells to the first antimicrobial agent or mixture and the salt or salt mixture. The candidate gram classifications could be regarded as preliminary or tentative gram classifications as determined on the respective phenotype characteristic responses. The two candidate gram classifications may then be compared to each other and if they both indicate the same gram classification, such as both indicate gram positive or both indicate gram negative, then the gram classification for the bacterial cells is determined to be the gram classification as indicated by both candidate gram classifications.

In another embodiment, if the first candidate gram classification and the second candidate gram classification indicate different gram classifications then step S32 in Fig. 7 preferably comprises determining the gram classification for the bacterial cells as a predefined gram classification.

In this embodiment, the two candidate gram classifications indicate different gram classifications, i.e., one indicates gram positive and the other indicates gram negative, or one or both candidate gram classifications indicate indefinable gram classification. In such a case, the bacterial cells will be assigned a predefined gram classification in step S32.

In an embodiment, this predefined gram classification is gram positive. In another preferred embodiment, this predefined gram classification is gram negative.

Hence, in a preferred embodiment, the gram classification for the bacterial cells is determined to be equal to the gram classification as indicated by the first and second candidate gram classifications if they both indicate the same gram classification (gram positive or gram negative) and otherwise is determined to be the predefined gram classification, preferably gram negative.

In an embodiment, the method as shown in Fig. 2 comprises an additional step S3. This step S3 comprises exposing bacterial cells from the biological sample to a third antimicrobial agent or mixture. The method then continues to step S5, or to any of the optional step S4.

In this embodiment, steps S1, S2 and S3 can be performed serially in any order but are preferably performed at least partly in parallel. In this latter preferred case, bacterial cells from the biological sample are exposed to the first antimicrobial agent or mixture in parallel with exposing other bacterial cells from the biological sample to the second antimicrobial agent or mixture and/or exposing other bacterial cells from the biological sample to the salt or salt mixture and other bacterial cells from the biological sample to the third antimicrobial agent or mixture.

In an embodiment, optional step S11 in Fig. 3 preferably comprises determining the genus classification for the bacterial cells based on the phenotype characteristic response of the bacterial cells to the second antimicrobial agent or mixture and a phenotype characteristic response of the bacterial cells to the third antimicrobial agent or mixture, and preferably based on the phenotype characteristic response of the bacterial cells to the first antimicrobial agent or mixture.

Hence, in this particular embodiment, the phenotypic characteristic responses of the bacterial cells to the second and third antimicrobial agents or mixtures, and preferably also the first antimicrobial agent or mixture, are used to determine the genus classification in step S11, such as to determine whether the bacterial cells are of a first genus or of a second genus.

In another embodiment, step S11 comprises determining the genus classification of the bacterial cells based on the phenotype characteristic response of the bacterial cells to the first antimicrobial agent or mixture, the phenotype characteristic response of the bacterial cells to the second antimicrobial agent or mixture and the phenotype characteristic response of the bacterial cells to the third antimicrobial agent or mixture if valid data of the phenotype characteristic responses of the bacterial cells to the first, second and third antimicrobial agents or mixtures are available. This embodiment also comprises determining the genus classification in step S11 for the bacterial cells based on the phenotype characteristic response of the bacterial cells to the salt or salt mixture and the phenotype characteristic response of the bacterial cells to one of the first, second and third antimicrobial agents or mixtures if valid data of the phenotype characteristic response of the bacterial cells to merely one of the first, second and third antimicrobial agents or mixtures is available.

Fig. 8 is a flow chart illustrating a subroutine of step S11 in Fig. 3 according to an embodiment. The method continues from step S10 in Fig. 3. A next step S40 comprises determining a first candidate genus classification for the bacterial cells based on the phenotype characteristic response of the bacterial cells to the second antimicrobial agent or mixture. Correspondingly, step S41 comprises determining a second candidate genus classification for the bacterial cells based on the phenotype characteristic response of the bacterial cells to the third antimicrobial agent or mixture.

These steps S40 and S41 can be performed serially in any order or at least partly in parallel.

A next step S42 comprises, in an embodiment, determining the genus classification for the bacterial cells as a genus classification indicated by the first candidate genus classification and the second candidate genus classification if both the first candidate genus classification and the second candidate genus classification indicate the same genus classification.

This embodiment thereby determines a respective candidate genus classification for the bacterial cells based on the phenotype characteristic response of the bacterial cells to the second and third antimicrobial agent or mixture. The candidate gram classifications could be regarded as preliminary or tentative genus classifications as determined on the respective phenotype characteristic responses. The two candidate genus classifications may then be compared to each other and if they both indicate the same genus classification, i.e., both indicates the same genus, then the genus classification for the bacterial cells is determined to be the genus classification as indicated by both candidate genus classifications.

In another embodiment, if the first candidate genus classification and the second candidate genus classification indicate different genus classifications then a phenotype characteristic response of the bacterial cells to the salt or salt mixture is used to determine the genus classification.

Hence, in this embodiment, the method comprises both step S3 and exposing bacterial cells to a salt or salt mixture in step S2 in Fig. 2. Step S42 in Fig. 8 then comprises determining the genus classification for the bacterial cells based on a phenotype characteristic response of the bacterial cells to the salt or salt mixture if the first candidate genus classification and the second candidate genus classification indicate different genus classifications.

In this embodiment, the two candidate genus classifications indicate different genus classifications, i.e., one indicates one genus and the other indicates another genus, or one or both candidate genus classifications indicate indefinable genus classification. In such a case, the genus classification is determined for the bacterial cells in step S42 based on the phenotype characteristic response of the bacterial cells to the salt or salt mixture.

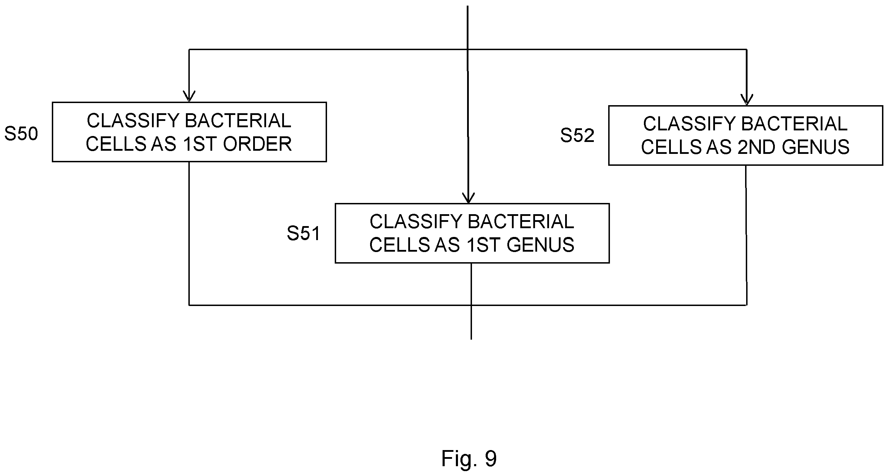

Fig. 9 is a flow chart illustrating a subroutine of the classifying step S12 in Fig. 3 according to an embodiment. In this embodiment, the bacterial cells are classified in step S50 as a first order if the gram classification is a first gram classification. Correspondingly, the bacterial cells are classified in step S51 as a first genus if the gram classification is a second gram classification and the genus classification is a first genus classification. Furthermore, the bacterial cells are classified in step S52 as a second genus if the gram classification is the second gram classification and the genus classification is a second genus classification.

This means that the gram and genus classifications as determined in steps S10 and S11 of Fig. 3 or in step S32 in Fig. 7 and step S42 in Fig. 8 are used in the embodiment as shown in Fig. 9 to determine whether the bacterial cells belong to a first order, a first genus or a second genus.

As an illustrative, but non-limiting, example of the embodiment shown in Fig. 8, the bacterial cells are classified Enterobacterales in step S50 if the gram classification is a gram negative. In this example, the bacterial cells are classified in step S51 as *Staphylococcus* if the gram classification is gram positive and the genus classification is *Staphylococcus.* In this example, step S52 comprises classifying the bacterial cells as *Enterococcus* if the gram classification gram positive and the genus classification is *Enterococcus.*

In this particular example, the candidate gram classifications determined in steps S30 and S31 of Fig. 7 thereby indicate gram positive or gram negative and the candidate genus classifications determined in steps S40 and S41 of Fig. 8 indicate *Staphylococcus* or *Enterococcus.*

The above presented example is in particular suitable for testing and classifying bacterial cells from a biological sample, in particular a urine sample, taken from a subject suffering from urine tract infection (UTI).

The first to third antimicrobial agent or mixture and the salt or salt mixture as used in steps S1 to S3 in Fig. 2, are preferably dissolved in a solvent, and in particular in a culture medium. In such a case, the same solvent, such as culture medium, is preferably used for all the antimicrobial agents or mixtures and the salt of salt mixtures.

In an embodiment, the method also comprises exposing bacterial cells from the biological sample to the solvent, such as culture medium. In such a case, step S5 in Fig. 2 preferably comprises classifying the bacterial cells from the biological sample at least based on (i) the phenotype characteristic response of the bacterial cells to the first antimicrobial agent or mixture, (iia) the phenotype characteristic response of the bacterial cells to the second antimicrobial agent or mixture and/or (iib) the phenotype characteristic response of the bacterial cells to the salt or salt mixture and (iii) a reference phenotype characteristic response of the bacterial cells to the solvent, such as culture medium.

As an example, a normalized phenotype characteristic response could be determined as a quotient between the phenotype characteristic response of the bacterial cells to the first or second antimicrobial agent or mixture or the salt or salt mixture and the reference characteristic response. This then means that step S5 in Fig. 2 comprises classifying the bacterial cells from the biological sample based on (i) a normalized phenotype characteristic response of the bacterial cells to the first antimicrobial agent or mixture and (iia) a normalized phenotype characteristic response of the bacterial cells to the second antimicrobial agent or mixture and/or (iib) a normalized phenotype characteristic response of the bacterial cells to the salt or salt mixture.

For instance, an impact parameter ranging from 0 to 1 could be calculated based on the normalized phenotype characteristic response. Such an impact parameter is then calculated as 1 - the normalized phenotype response, i.e., $1 - \dfrac{PCR_{treated}}{PCR_{control}}$ , wherein $PCR_{treated}$ represents the phenotype characteristic response of the bacterial cells treated by and thereby exposed to the first or second antimicrobial agent or mixture and $PCR_{control}$ represents the reference phenotype characteristic response of the bacterial cells exposed to the solvent, such a culture medium, lacking any antimicrobial agent or mixture.

As an example, a first impact parameter is calculated based on the phenotype characteristic response of the bacterial cells to the first antimicrobial agent or mixture and the reference characteristic response and a second impact parameter is calculated based on the phenotype characteristic response of the bacterial cells to the second antimicrobial agent or mixture and the reference characteristic response. In such an example, the classification of the bacterial cells in step S5 can be based on the first and second impact parameters.

The above-described embodiments of using reference characteristic response and calculating impact parameters can also be applied to determining the gram classification in step S10, determining the genus classification in step S11, determining the first candidate gram classification in step S30, determining the second candidate gram classification in step S31, determining the first candidate genus classification in step S40, and/or determining the second candidate genus classification in step S41. For instance, $PCR_{treated}$ as mentioned above could then represent the phenotype characteristic response of the bacterial cells treated by and thereby exposed to the third antimicrobial agent or mixture or exposed to the salt or salt mixture.

The exposure of bacterial cells to merely the solvent, such as culture medium, is preferably also of benefit for determining the at least one morphology characteristic of the bacterial cells in step S4 in Fig. 2. Hence, at least one image, but typically a plurality of images are taken of the cell channels 140 comprising captured bacterial cells exposed to the solvent, such as culture medium. The one or more images is or are then input into the neural network (deep learning model) as previously described herein in order to output a tentative classification of the bacterial cells as, preferably, rod-shaped (rods) or cocci-shaped (cocci).

In an embodiment, step S5 in Fig. 2 comprises classifying the bacterial cells at least based on the phenotype characteristic response of the bacterial cells to the first antimicrobial agent or mixture and the phenotype characteristic response of the bacterial cells to the second antimicrobial agent or mixture if valid data of the phenotype characteristic response of the bacterial cells to the second antimicrobial agent or mixture is available. In another embodiment, step S5 in Fig. 2 comprises classifying the bacterial cells at least based on the phenotype characteristic response of the bacterial cells to the first antimicrobial agent or mixture and the phenotype characteristic response of the bacterial cells to the salt or salt mixture if no valid data of the phenotype characteristic response of the bacterial cells to the second antimicrobial agent or mixture is available.

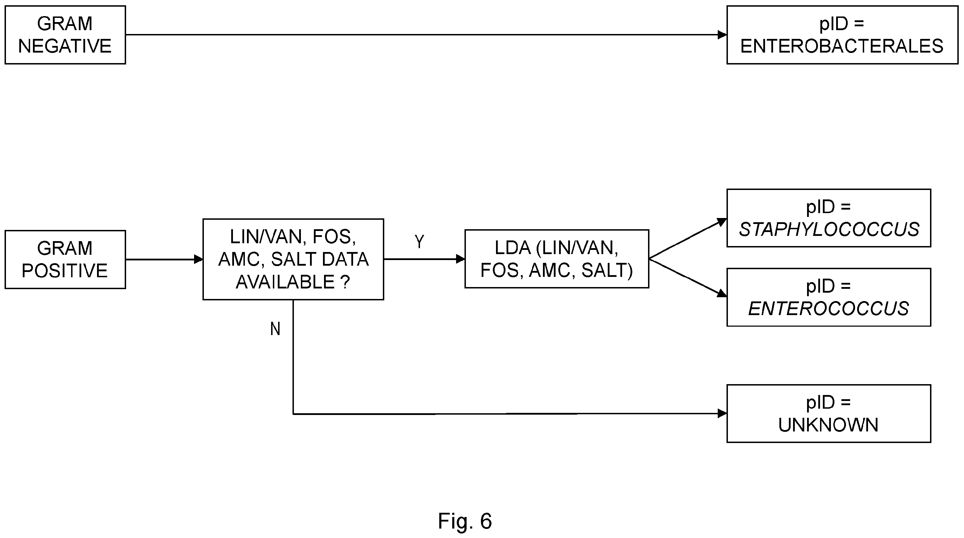

Fig. 6 schematically illustrate an embodiment of the classification. This embodiment is in particular adapted for classifying bacterial cells present in a biological sample, such as urine sample, taken from a subject suffering from UTI.

In this embodiment, if the gram classification, such as performed in accordance with Fig. 5, classifies the bacterial cells as gram negative, then the bacterial cells are classified as Enterobacterales. However, if the bacterial cells are instead classified in Fig. 5 as gram positive the classification is based on the response on the different treatments.

In this embodiment, bacterial cells from the biological sample are exposed to four different treatments. The first treatment comprises a combination of LIN and VAN in step S1 of Fig. 2, the second treatment comprises AMC in step S2 of Fig. 2, the third treatment comprises FOS in step S3 of Fig. 2 and the fourth treatment is a salt treatment with sodium chloride (NaCl) in step S2 of Fig. 2.

An optional validation step is first conducted to check whether valid LINNAN, FOS, AMC and/or salt data for performing the genus classification is available. In particular, this validation step is conducted to check whether valid data of at least two of LINNAN, FOS, AMC and salt data are available. If no such data is available, no genus classification can be done, and the bacterial cells are classified as being unknown. However, if valid data from at least two of the treatments is available, an LDA-based classification is performed. The LDA-based gram positive classification is performed by first calculating the antibiotic impact (reduction of normalized growth rate fitted to a logistic function) for each treatment (AMC, FOS, LINNAN) as well as the growth rate reduction in the salt treatment (not fitted data, but average normalized growth rate in a set time interval). LINNAN is a single treatment, but for AMC and FOS the impact is based on a "dose-response curve", a logistic curve fitted to the antibiotic impact at a set timepoint of all valid treatments of each antibiotic. The impact is then defined as the reduction in normalized growth rate at a given concentration for AMC and FOS based on the fitted curve. These impact values are then used as a vector, such as in the form [AMC impact, FOS impact, salt growth rate reduction, LIN/VAN impact]. If a variable is missing, it is removed from the vector, but the order of the remaining variables is preferably kept. If the dataset is complete, or only one variable of the variables AMC, FOS or LIN/VAN is missing, the salt growth rate reduction is removed from the vector, as it is generally the data with most noise.

For instance, assume that valid data from the AMC, FOS and LIN/VAN treatments are available. In such a case, the vector is defined as [AMC impact, FOS impact, LINNAN impact]. Correspondingly, assume that valid data from two of AMC, FOS and LIN/VAN treatments are available, such as AMC and LIN/VAN treatments, then the vector is defined as [AMC impact, LINNAN impact]. However, if valid data is available from merely one of the AMC, FOS and LIN/VAN treatments then also the data from the salt treatment is included in the vector, such as [salt growth rate reduction, LINNAN impact] for the case where valid LINNAN data is available but no AMC or FOS data are available.

Based on a large calibration dataset, eigenvectors of the data's covariance matrix (principal components) have been calculated that maximizes the variance of the projected data. The vector is then projected down on the eigenvector by calculating the dot product of the two vectors. The resulting scalar is then used to determine the classification (pID) by comparing it to a threshold value. The bacterial cells are then classified as either *Staphylococcus* or *Enterococcus.*

For instance, if the vector [AMC impact, FOS impact, LIN/VAN impact] would be [0.1, 0.1, 0.9], and the eigenvector would be [0.7, 0.7, -0.2], the resulting scalar would be the dot product of the two vectors: [0.1, 0.1, 0.9] • [0.7, 0.7, -0.2] = -0.04. If this value is below a defined threshold value, such as 0.5, the classification is, in this example, *Enterococcus.*

Hence, in an embodiment, classifying the bacterial cells comprises classifying the bacterial cells at least based on (i) the phenotype characteristic response of the bacterial cells to the first antimicrobial agent or mixture, and (iia) the phenotype characteristic response of the bacterial cells to the second antimicrobial agent or mixture and/or (iib) the phenotype characteristic response of the bacterial cells to the salt or salt mixture if the bacterial cells have a predefined gram classification, preferably gram positive.

In a particular embodiment, classifying the bacterial cells comprises classifying the bacterial cells as *Staphylococcus* or *Enterococcus.*

In an embodiment, the method comprises step S3 as shown in Fig. 2. This step S3 comprises exposing bacterial cells from the biological sample to a third antimicrobial agent or mixture. In such an embodiment, classifying the bacterial cells comprises classifying the bacterial cells based on at least two of the phenotype characteristic response of the bacterial cells to the first antimicrobial agent or mixture, the phenotype characteristic response of the bacterial cells to the second antimicrobial agent or mixture, the phenotype characteristic response of the bacterial cells to the salt or salt mixture, and a phenotype characteristic response of the bacterial cells to the third antimicrobial agent or mixture.

In an embodiment, classifying the bacterial cells comprises classifying the bacterial cells based on the phenotype characteristic responses of the bacterial cells to at least two of the first, second and third antimicrobial agents or mixtures if valid data of the phenotype characteristic responses of the bacterial cells to the at least two of the first, second and third antimicrobial agents or mixtures are available. In another embodiment, classifying the bacterial cells comprises classifying the bacterial cells based on the phenotype characteristic response of the bacterial cells to the salt or salt mixture and the phenotype characteristic response of the bacterial cells to one of the first, second and third antimicrobial agents or mixtures if valid data of the phenotype characteristic response of the bacterial cells to merely one of the first, second and third antimicrobial agents or mixtures is available.

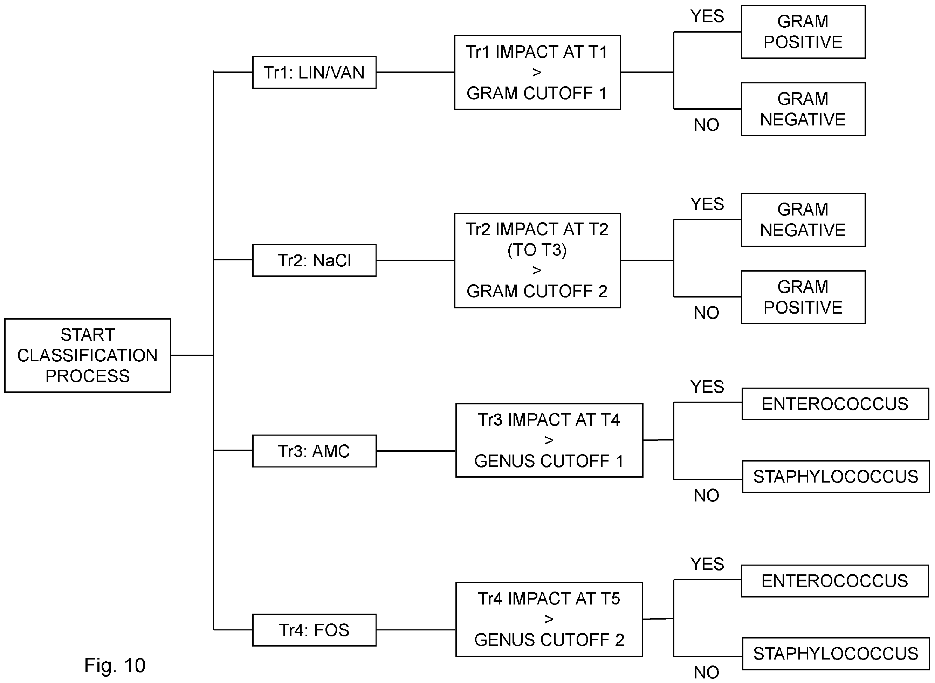

Figs. 10 to 13 schematically illustrate a subroutine of the classification step S5 in Fig. 2 according to an embodiment. This embodiment is in particular adapted for classifying bacterial cells present in a biological sample, such as urine sample, taken from a subject suffering from UTI.

In this embodiment, bacterial cells from a biological sample are exposed to four different treatments (Tr1 to Tr4). The first treatment Tr1 comprises a combination of linezolid (LIN) and vancomycin (VAN), the second treatment Tr2 comprises a salt treatment with sodium chloride (NaCl), the third treatment Tr3 comprises amoxicillin and clavulanic acid (AMC) and the fourth treatment Tr4 comprises fosfomycin (FOS). The combination of LIN/VAN in the first treatment Tr1 is generally effective against gram-positive bacteria but has no or little antibacterial activity against gram-negative bacteria. Correspondingly, salt treatment Tr2 using NaCl is generally effective against gram-negative bacteria, but has less antibacterial activity against gram-positive bacteria, in particular little or no impact against *Staphylococcus.* AMC used in the third treatment Tr3 has broad antibacterial activity against both gram-positive and gram-negative bacteria. It, though, generally has slower antibacterial activity against *Staphylococcus* as compared to against *Enterococcus.* FOS used in the fourth treatment Tr4 has a broad antibacterial activity against both gram-positive and gram-negative bacteria. It, though, generally has a slower antibacterial activity against *Staphylococcus* as compared to against *Enterococcus.* Bacterial cells from the biological sample are preferably exposed to respective treatment in parallel. In this example, a respective impact parameter is determined for each treatment after a respective treatment period (T1 to T5) and then compared to a respective cutoff or threshold value. The respective treatment periods T1 to T5 could be the same but are typically different and adapted to the particular antimicrobial agent or mixture or salt or salt mixture.

In more detail, the Tr1 impact parameter is compared to a first gram cutoff or threshold value and if it exceeds the first gram cutoff or threshold value the first candidate gram classification for the bacterial cells is set as gram positive otherwise it is set as gram negative. Correspondingly, the Tr2 impact parameter is compared to a second gram cutoff or threshold value and if it exceeds the second gram cutoff or threshold value the second candidate gram classification for the bacterial cells is set as gram negative otherwise it is set as gram positive.

The Tr3 impact parameter is compared to a first genus cutoff or threshold value and if it exceeds the first genus cutoff or threshold value the first candidate genus classification for the bacterial cells is set as *Enterococcus* otherwise it is set *Staphylococcus.* Correspondingly, the Tr4 impact parameter is compared to a second genus cutoff or threshold value and if it exceeds the second genus cutoff or threshold value the second candidate genus classification for the bacterial cells is set as *Enterococcus* otherwise it is set *Staphylococcus.*

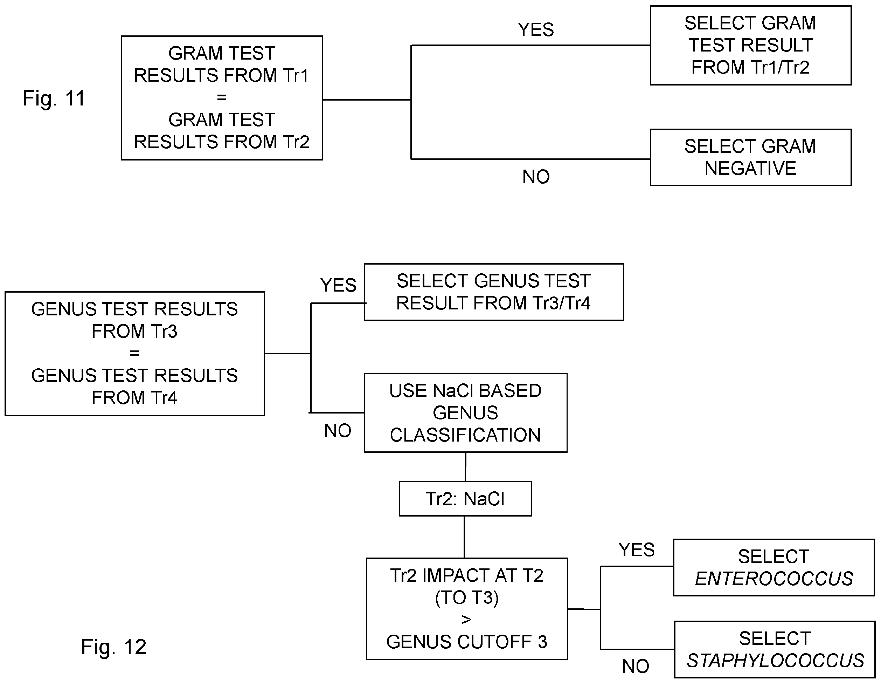

Fig. 11 schematically illustrates the gram type classification of the classification process according to an embodiment. In this embodiment, the gram classification for the bacterial cells is selected as the gram test result from the first treatment Tr1 and the second treatment Tr2 if the gram test results from these two treatments are the same, i.e., both indicate gram positive or gram negative. If the two gram test results are not the same then the gram classification is selected to be a predefined gram classification, in this case gram negative. Using gram negative as the predefined gram classification is in particular adapted for analyzing a biological sample, such as urine sample, taken from a subject suffering from UTI. The reason being that such UTI are more commonly caused by gram-negative bacteria as compared to gram-positive bacteria and in the rare case of disagreement in classification by the two treatments Tr1 and Tr2 then gram negative is preferably selected.

Fig. 12 schematically illustrates the genus classification of the classification process according to an embodiment. In this embodiment, the genus classification for the bacterial cells is selected as the genus test result from the third treatment Tr3 and the fourth treatment Tr4 if the genus test results from these two treatments are the same, i.e., both indicate *Staphylococcus* or *Enterococcus*. If the test results from the third and fourth treatments T3, Tr4 are not the same, a salt (NaCl) based genus classification will instead be used, i.e., the second treatment Tr2. Generally, *Enterococcus* bacteria are less salt tolerant as compared to *Staphylococcus* bacteria. Hence, the salt treatment Tr2 could be used as a confirmative treatment verification if the third and fourth treatments Tr3, Tr4 gave different genus test results. The Tr2 impact parameter is compared to a third genus cutoff or threshold value and if it exceeds the third genus cutoff or threshold value the genus classification for the bacterial cells is set as *Enterococcus* otherwise it is set *Staphylococcus.*

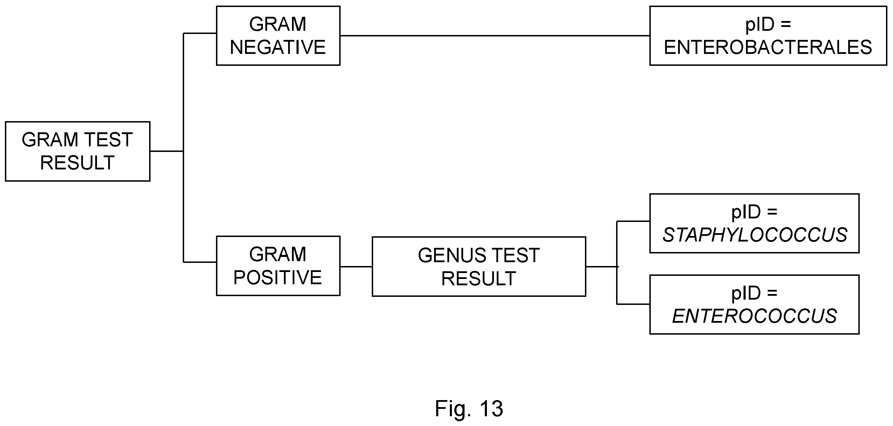

Fig. 13 schematically illustrates the final bacterial classification of the classification process according to an embodiment. In this embodiment, the gram classification from Fig. 11 is first analyzed. If it indicates gram negative then the bacterial cells are classified as being Enterobacterales. However, if the gram classification instead indicates gram positive, the genus classification as determined in Fig. 12 is used to classify the bacterial cells either as *Staphylococcus* or *Enterococcus.*

In an embodiment, a method for classifying bacterial cells from a biological sample comprises monitoring bacterial cells from the biological sample at multiple time instances while the bacterial cells are not exposed to any antimicrobial agent or mixture. The method also comprises exposing bacterial cells from the biological sample to an antimicrobial agent or mixture. The method further comprises determining a morphology classification of the bacterial cells based on monitoring the bacterial cells at the multiple time instances. The method additionally comprises classifying the bacterial cells from the biological sample based on the morphology classification and a phenotype characteristic response of the bacterial cells to the antimicrobial agent or mixture.

In an embodiment, monitoring the bacterial cells comprises monitoring bacterial cells from the biological sample captured in multiple cells channels while the bacterial cells are not exposed to any antimicrobial agent or mixture. In this embodiment, determining the morphology classification comprises, for each cell channel of the multiple channels, determining, at each time instance of the multiple time instances, a tentative channel-specific morphology classification of the bacterial cells captured in the cell channel, determining a channel-specific morphology classification of the bacterial cells captured in the cell channel based on the tentative channel-specific morphology classifications, and determining the morphology classification of the bacterial cells based on the channel-specific morphology classifications.

In an embodiment, determining the tentative channel-specific morphology classification comprises determining, at each time instance of the multiple time instances, the tentative channel-specific morphology classification of the bacterial cells based on an image of the bacterial cells in the cell channel and a computer-implemented morphology classification model trained for classifying bacterial cells based on images of the bacterial cells.

In an embodiment, determining the channel-specific morphology classification comprises determining the channel-specific morphology classification as a first channel-specific morphology classification if at least a minimum portion of the tentative channel-specific morphology classifications indicates a first classification, as a second channel-specific morphology classification if the at least a minimum portion of the tentative channel-specific morphology classifications indicates a second classification, and otherwise as indefinite. In this embodiment, determining the morphology classification comprises determining the morphology classification of the bacterial cells based on a quotient between the number of cells channels classified as the first classification and the number cell channels classified as the second classification.

In an embodiment, classifying the bacterial cells comprises determining a gram classification of the bacterial cells from the biological sample based on the morphology classification and the phenotype characteristic response of the bacterial cells to the antimicrobial agent or mixture and determining a genus classification of the bacterial cells from the biological sample at least partly based on the gram classification.

In an embodiment, determining the genus classification comprises determining the genus classification of the bacterial cells as a first genus if the gram classification indicates a first gram classification.

In an embodiment, exposing the bacterial cells comprises exposing, for each set of multiple cell channels of multiple sets of multiple cell channels, bacterial cells from the biological sample captured in the set of multiple cell channels to a respective antimicrobial agent or mixture. In this embodiment, determining the genus classification comprises determining the genus classification based on phenotypic characteristic responses of the bacterial cells to the respective antimicrobial agents or mixtures if the gram classification indicates a second gram classification.

In an embodiment, determining the genus classification comprises determining, for each antimicrobial agent or mixture of the respective antimicrobial agents or mixtures, any reduction of growth rate of the bacterial cells in response to exposure to the antimicrobial agent or mixture, and determining the genus classification based on the reductions of growth rate of the bacterial cells in response to exposure to the respective antimicrobial agents or mixtures if the gram classification indicates the second gram classification.

In an embodiment, determining the genus classification comprises calculating a dot product of a vector of the reductions of grow rate of the bacterial cells and an eigenvector of a covariance matrix of growth rate calibration data to get a scalar value and determining the genus classification based on a comparison of the scalar value and threshold value.

In an embodiment, the method further comprises determining a validity of the phenotype characteristic response of the bacterial cells to the antimicrobial agent or mixture. In this embodiment, classifying the bacterial cells comprises classifying the bacterial cells from the biological sample based on the morphology classification but not the phenotype characteristic response of the bacterial cells to the antimicrobial agent or mixture if the phenotype characteristic response of the bacterial cells to the antimicrobial agent or mixture is determined to be not valid.

In an embodiment, the method further comprises determining a validity of the morphology classification of the bacterial cells. In this embodiment, exposing the bacterial cells comprises exposing, for each set of multiple cell channels of multiple sets of multiple cell channels, bacterial cells from the biological sample captured in the set of multiple cell channels to a respective antimicrobial agent or mixture. In this embodiment, classifying the bacterial cells comprises classifying the bacterial cells from the biological sample based on phenotype characteristic responses of the bacterial cells to the respective antimicrobial agents or mixtures but not the morphology classification of the bacterial cells if the morphology classification of the bacterial cells is determined to be not valid.

In an embodiment, classifying the bacterial cells comprises determining a gram classification for the bacterial cells based on a phenotype characteristic response of the bacterial cells to a first antimicrobial agent or mixture of the respective antimicrobial agents or mixtures, determining a genus classification for the bacterial cells based on a phenotype characteristic response of the bacterial cells to a second antimicrobial agent or mixture of the respective antimicrobial agents or mixtures, and classifying the bacterial cells based on the gram classification and the genus classification.

In an embodiment, the method further comprises exposing bacterial cells from the biological sample to a salt or salt mixture. In this embodiment, determining the gram classification comprises determining the gram classification for the bacterial cells based on the phenotype characteristic response of the bacterial cells to the first antimicrobial agent or mixture and a phenotype characteristic response of the bacterial cells to the salt or salt mixture.

In an embodiment, determining the gram classification comprises determining a first candidate gram classification for the bacterial cells based on the phenotype characteristic response of the bacterial cells to the first antimicrobial agent or mixture, determining a second candidate gram classification for the bacterial cells based on the phenotype characteristic response of the bacterial cells to the salt or salt mixture, and determining the gram classification for the bacterial cells as a gram classification indicated by the first candidate gram classification and the second candidate gram classification if both the first candidate gram classification and the second candidate gram classification indicate the same gram classification and determining the gram classification for the bacterial cells as a predefined gram classification if the first candidate gram classification and the second candidate gram classification indicate different gram classifications.

In an embodiment, the method further comprises exposing bacterial cells from the biological sample to a third antimicrobial agent or mixture of the respective antimicrobial agents or mixtures. In this embodiment, determining the genus classification comprises determining the genus classification for the bacterial cells based on the phenotype characteristic response of the bacterial cells to the second antimicrobial agent or mixture and a phenotype characteristic response of the bacterial cells to the third antimicrobial agent or mixture.

In an embodiment, determining the genus classification comprises determining a first candidate genus for the bacterial cells based on the phenotype characteristic response of the bacterial cells to the second antimicrobial agent or mixture, determining a second candidate genus classification for the bacterial cells based on the phenotype characteristic response of the bacterial cells to the third antimicrobial agent or mixture, and determining the genus classification for the bacterial cells as a genus classification indicated by the first candidate genus classification and the second candidate genus classification if both the first candidate genus classification and the second candidate genus classification indicate the same genus classification.

In an embodiment, the method further comprises exposing the bacterial cells from the biological sample to a salt or salt mixture. In this embodiment, determining the genus classification comprises determining a first candidate genus for the bacterial cells based on the phenotype characteristic response of the bacterial cells to the second antimicrobial agent or mixture, determining a second candidate genus classification for the bacterial cells based on the phenotype characteristic response of the bacterial cells to the third antimicrobial agent or mixture, and determining the genus classification for the bacterial cells based on a phenotypic characteristic response of the bacterial cells to the salt or salt mixture if the first candidate genus classification and the second candidate genus classification indicate different genus classifications.

In an embodiment, classifying the bacterial cells comprises classifying the bacterial cells as a first order if the gram classification is a first gram classification, classifying the bacterial cells as a first genus if the gram classification is a second gram classification and the genus classification is a first genus classification, and classifying the bacterial cells as a second genus if the gram classification is the second gram classification and the genus classification is a second genus classification.

In an embodiment, classifying the bacterial cells comprises classifying the bacterial cells as Enterobacterales if the gram classification is a gram negative, classifying the bacterial cells as *Staphylococcus* if the gram classification is gram positive classification and the genus classification is *Staphylococcus,* and classifying the bacterial cells as *Enterococcus* if the gram classification gram positive and the genus classification is *Enterococcus.*

The classification of the bacterial cells from the biological sample as determined by the method shown in Fig. 2 including embodiments thereof shown in Figs. 3 to 13 can, as mentioned in the foregoing, be used to determine or select a CBP for a test antimicrobial agent. Hence, the classification of the bacterial cells, such as in terms of Enterobacterales, *Staphylococcus* or *Enterococcus* for the example above, can be used as input to determine or select a CBP for one or more antimicrobial agents from published lists of CBP values, such as available from EUCAST or CLSI.

The classification of the bacterial cells can also be performed as a part of an analysis of the bacterial cells as disclosed in Fig. 5.

Fig. 14 is a flow chart illustrating a method for analyzing bacteria. The method comprises exposing bacterial cells from a biological sample to a test antimicrobial agent in step S60. A next step S61 corresponds to the classification of bacterial cells as previously described herein with reference to Figs. 2 to 13. Hence, this step S61 comprises at least steps S1, S2 and S5 of Fig. 2, but preferably also steps S3 and S4 of Fig. 2. Bacterial cells from the biological sample are exposed to a first antimicrobial agent or mixture in step S1 of Fig. 2 and to a second antimicrobial agent or mixture and/or to a salt or salt mixture in step S2 of Fig. 2.

Steps S60, S1 and S2 can be performed serially in any order but are preferably performed at least partly in parallel. In this latter preferred case, bacterial cells from the biological sample are exposed to the test antimicrobial agent in parallel with exposing other bacterial cells from the biological sample to the first antimicrobial agent or mixture and other bacterial cells from the biological sample to the second antimicrobial agent or mixture and/or other bacterial cells from the biological sample to the salt or salt mixture.

A next step S5 of Fig. 2 comprises classifying the bacterial cells at least based on (i) a phenotype characteristic response of the bacterial cells to the first antimicrobial agent or mixture and (iia) a phenotype characteristic response of the bacterial cells to the second antimicrobial agent or mixture and/or (iib) a phenotype characteristic response of the bacterial cells to the salt or salt mixture.

Step S61, thus, preferably corresponds to steps S1, S2 and S5 in Fig. 2 and may, thus, be performed as described in the foregoing with reference to Fig. 2 or indeed any of the embodiments described in the foregoing with reference to any or all of Figs. 3 to 13. Hence, in an embodiment, the method as shown in Fig. 14 may also comprise exposing bacterial cells to a third antimicrobial agent or mixture corresponding to step S3 in Fig. 2, exposing bacterial cells to a salt or salt mixture corresponding to step S2 in Fig. 2, and/or determining at least one morphology characteristic of the bacterial cells corresponding to step S4 in Fig. 2.

A next step S62 comprises determining a CBP for the test antimicrobial agent used in step S60 based on a result of classifying the bacterial cells in step S5 of Fig. 2. The method also comprises determining a MIC of the test antimicrobial agent based on a phenotype characteristic response of the bacterial cells to the test antimicrobial agent. The MIC is then compared with the CBP in step S64.

In an embodiment, the method comprises an additional step S65 as shown in Fig. 15. This step S65 comprises determining whether bacterial cells are susceptible to the test antimicrobial agent based on the comparison between the MIC and CBP.

In more detail, if the MIC as determined in step S63 is below the CBP determined or selected in step S62, the bacterial strain is classified as susceptible (S) to the test antimicrobial agent, whereas if the MIC is above the CBP, the bacterial strain is classified as resistant (R) to the test antimicrobial agent.

As mentioned in the foregoing, in some cases two CBP values are available for an antimicrobial agent and an order/family/genus/species, a susceptibility CBP value and a resistance CBP value, and could thereby be determined for the bacterial cells in step S62. In such a case, the MIC value as determined in step S63 could be compared to the susceptibility and resistance CBP values determined in step S62 to determine whether the bacterial cells are susceptible (S), resistant (R) or of intermediate (I) phenotype regarding the test antimicrobial agent.

The discussion above with regard to exposing bacterial cells from the biological sample to a solvent, such as culture medium, and determining and using a reference phenotype characteristic response and calculating respective impact parameters also apply to the analyzing method in Figs. 14 and 15.

The analyzing method in Fig. 14 is not limited to testing a single test antimicrobial agent. In clear contrast, multiple different test antimicrobial agents may be tested, preferably in parallel. For instance, in an embodiment, step S60 comprises exposing bacterial cells from the biological sample to a first test antimicrobial agent and exposing bacterial cells from the biological sample to a second test antimicrobial agent. In this embodiment, step S62 comprises determining a first CBP for the first antimicrobial agent and a second CBP for the second antimicrobial agent. Step S63 comprises, in this embodiment, determining a first MIC of the first test antimicrobial agent based on the phenotype characteristic response of the bacterial cells to the first test antimicrobial agent and determining a second MIC concentration of the second test antimicrobial agent based on the phenotype characteristic response of the bacterial cells to the second test antimicrobial agent. The first MIC is then compared to the first CBP and the second MIC is compared to the second CBP in step S64.

The above-described approach may also be extended to testing more than two different test antimicrobial agents.

In the above-described embodiments, bacterial cells may be exposed to a first to third antimicrobial agent or mixture, a test antimicrobial agent or a salt or salt mixture at a single respective concentration in steps S1, S2, S3, S60, S61. Alternatively, bacterial cells from the biological sample may be exposed to multiple different concentrations of any or all of the first to third antimicrobial agent or mixture, the test antimicrobial agent or the salt or salt mixture in steps S1, S2, S3, S60, S61.

In the former case, the phenotype characteristic response of the bacterial cells to a respective single concentration of the first, second or third antimicrobial agent, or test antimicrobial agent or the salt or salt mixture is available and used in steps S5, S10, S11, S22, S25, S30, S31, S40, S41, S63. In the latter case, multiple phenotype characteristic responses of the bacterial cells to the respective multiple concentrations of the first, second or third antimicrobial agent, or test antimicrobial agent or the salt or salt mixture are available and can be used in steps S5, S10, S11, S22, S25, S30, S31, S40, S41, S63.

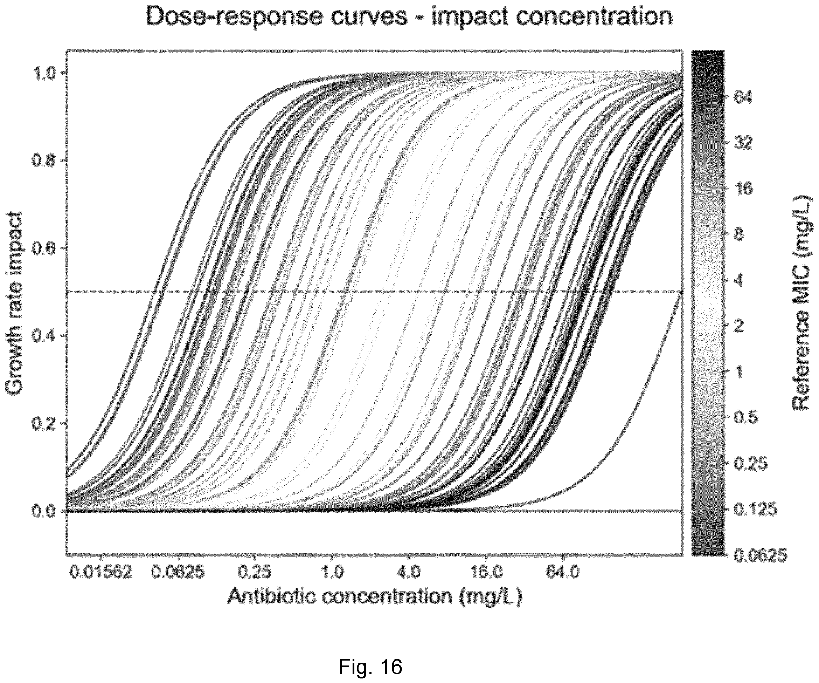

As an illustrative example, dose response curves as shown in Fig. 16 could be generated by mapping or plotting the growth rate impact versus concentration of the relevant antimicrobial agent or mixture or the salt or salt mixture. In an embodiment, an impact parameter in the form of growth rate impact is a normalized growth rate parameter for the bacterial cells and represents 1 - average growth rate of treated bacterial cells normalized by average growth rate of untreated bacterial

$$GRI = 1 - \frac{\overline{GR}_{treated}}{\overline{GR}_{control}}$$

cells, i.e., , wherein GRI represents growth rate impact, $\overline{GR}_{treated}$ represents average growth rate of treated bacterial cells and $\overline{GR}_{control}$ represents average growth rate of untreated bacterial cells, i.e., reference or control bacterial cells that have not been exposed to the particular antimicrobial agent or mixture or the salt or salt mixture but merely the solvent, such as culture medium.

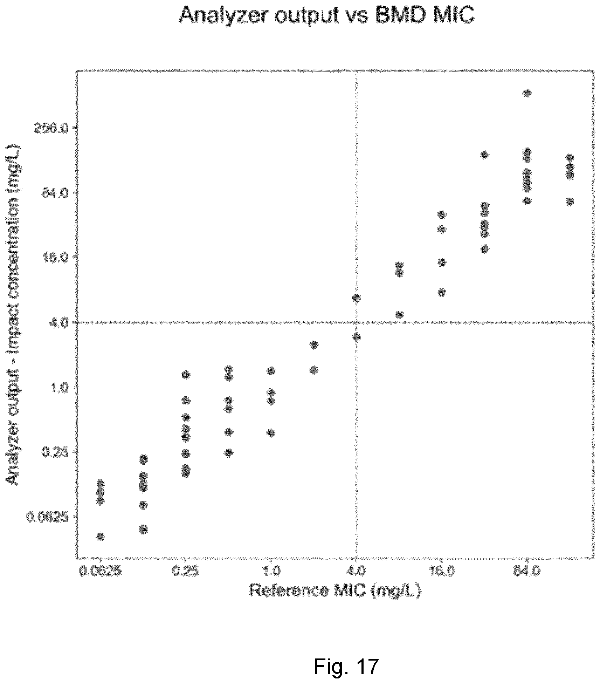

In more detail, Fig. 16 shows unique bacterial isolates being tested in separate microfluidic devices. These isolates can have different MICs (degree of susceptibility to an antibiotic) measured by a reference method and that is given by the coding of the line to the right of the diagram. Data in Fig. 16 shows how the growth rate impact (y-axis) of each isolate is changing as a function of concentration of the antibiotics applied within the same microfluidic device (x-axis). The intercept of the dotted line (y=0.5) with each curve corresponds to an impact concentration measurement, which is calibrated to provide a prediction of the MIC. Hence, an impact concentration can then be calculated from the intercept of the dose response curves with a pre-determined threshold as indicated by the hashed line in Fig. 16. As is further shown in Fig. 17, there is a correlation between the impact concentration and reference MIC values as determined using traditional culturing-based MIC methods.

In a particular embodiment, bacterial cells from the biological sample are exposed to at least one concentration, preferably one concentration, of the first antimicrobial agent or mixture in step S1, S61, bacterial cells from the biological sample are exposed to at least one concentration, preferably multiple concentrations, such as four to six concentrations, of the second antimicrobial agent or mixture in step S2, S61, bacterial cells from the biological sample are exposed to at least one concentration, preferably one concentration, of the salt or salt mixture in step S4, and bacterial cells from the biological sample are exposed to at least one concentration, preferably multiple concentrations, such as four to six concentrations, of the third antimicrobial agent or mixture in step S3. Correspondingly, bacterial cells from the biological sample are exposed to at least one concentration, preferably multiple concentrations, such as four to six concentrations, of the test antimicrobial agent in step S60.

In an embodiment, see Fig. 21, step S60 comprises exposing bacterial cells captured in a test set or sets 130 of cell channels 140 having a respective channel restriction 145 configured to restrict bacterial cells entering the cell channels 140 from leaving the cell channels 140 to the test antimicrobial agent. In this embodiment, step S61 comprises exposing bacterial cells captured in a first set or sets 130 of cell channels 140 having a respective channel restriction 145 configured to restrict bacterial cells entering the cell channels 140 from leaving the cell channels 140 to the first antimicrobial agent or mixture. Furthermore, step S62 comprises exposing bacterial cells captured in a second set or sets 130 of cell channels 140 having a respective channel restriction 145 configured to restrict bacterial cells entering the cell channels 140 from leaving the cell channels 140 to the second antimicrobial agent or mixture and/or exposing bacterial cells captured in a fourth set or sets 130 of cell channels 140 having a respective channel restriction 145 configured to restrict bacterial cells entering the cell channels 140 from leaving the cell channels 140 to the salt or salt mixture.

This embodiment could be extended also for the third antimicrobial agent or mixture. In such a case, step S3 in Fig. 2 comprises exposing bacterial cells captured in a third set or sets 130 of cell channels 140 having a respective channel restriction 145 configured to restrict bacterial cells entering the cell channels 140 from leaving the cell channels 140 to the third antimicrobial agent or mixture. In an embodiment, step S4 in Fig. 2 comprises exposing bacterial cells captured in a fifth set or sets 130 of cell channels 140 having a respective channel restriction 145 configured to restrict bacterial cells entering the cell channels 140 from leaving the cell channels 140 to a solvent, preferably a culture medium, and taking at least one image of bacterial cells captured in the fifth set or sets 130 of cell channels 140. The solvent, preferably culture medium, mentioned above is preferably the same solvent, preferably culture medium, in which the first to third antimicrobial agents or mixtures and the salt or salt mixtures are dissolved or dispersed.

The different sets of cell channels 140 are formed as separate compartments in a microfluidic chip 10.

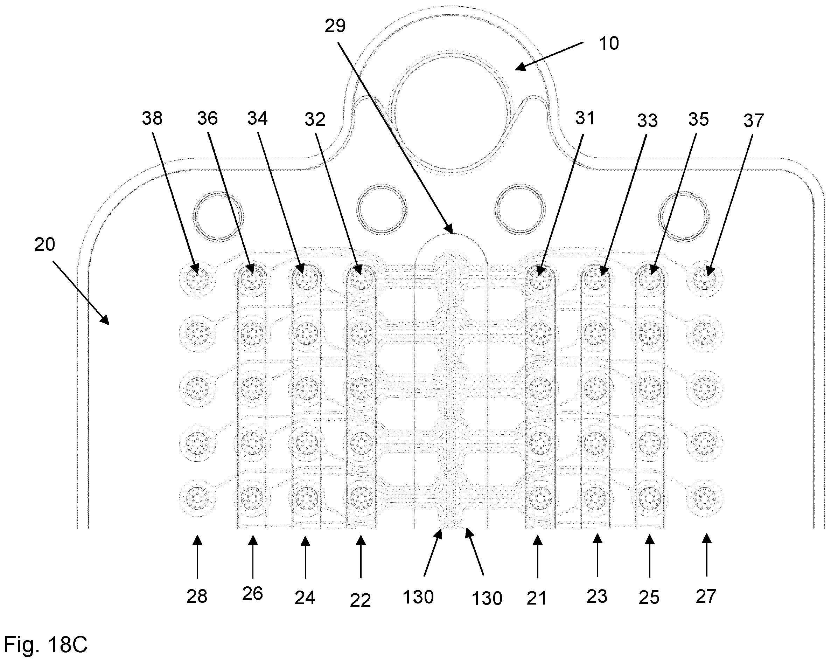

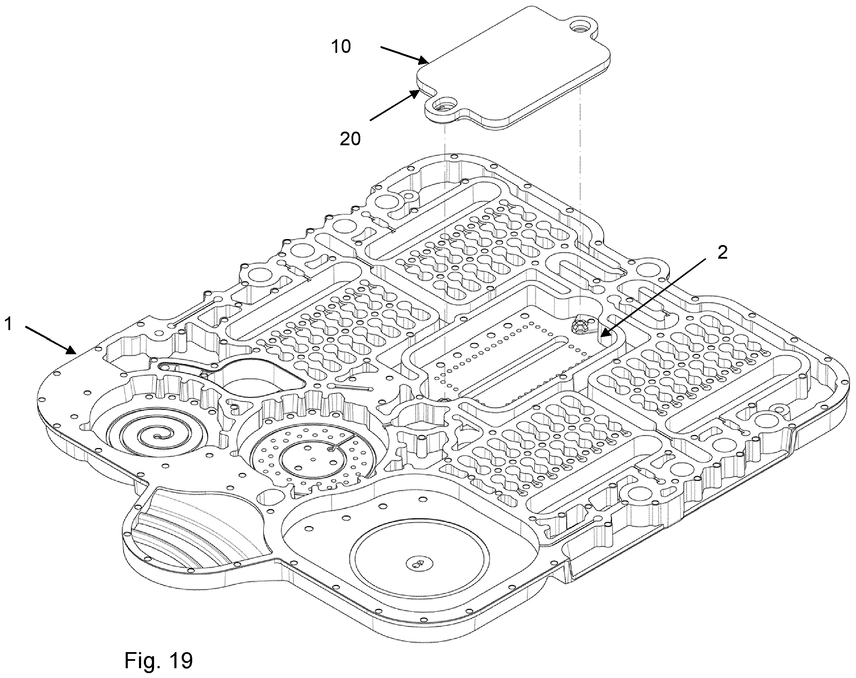

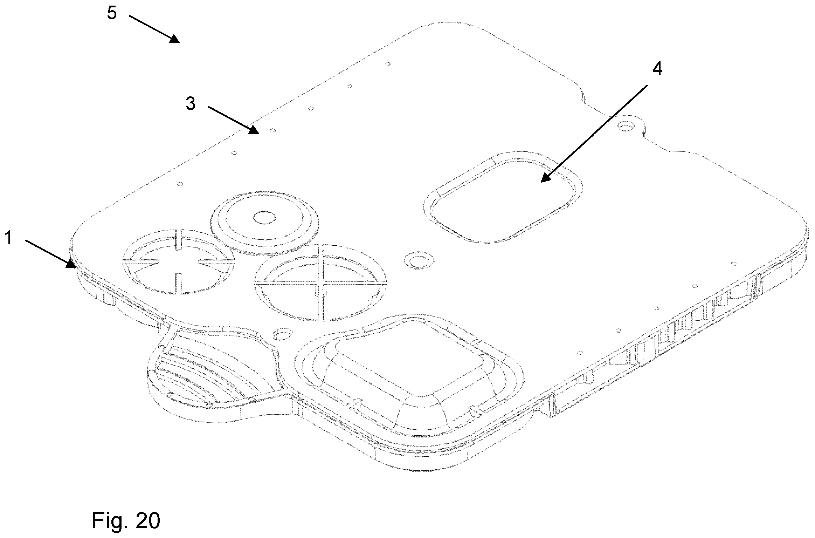

Hence, the different sets 130 of cell channels 140 are preferably present in a microfluidic chip 10 as shown in Figs. 18A to 18C. In particular Fig. 18C indicates that the microfluidic chip 10 may contain a plurality of such sets 130 of cell channels 140. The microfluidic chip 10, preferably attached to a chip carrier 20, is then inserted into a chip chamber 2 of a substrate 1 of a microfluidic device 5 as shown in Figs. 19 and 20.

In an embodiment, the method in Fig. 2 or 14 comprises loading a biological sample comprising bacterial cells into cell channels 140 of a microfluidic chip 10 having a plurality of sets 130 of cell channels 140, a plurality of input channels 110 and a plurality of output channels 120. As is more clearly shown in Fig. 21, each input channel 110 is in fluid communication with a respective output channel 120 through the cell channels 140 of a respective set 130 of cell channels 140. The cell channels 140 comprise a respective channel restriction 145 configured to restrict bacterial cells entering the cell channels 140 from reaching an output channel 120. The respective channel restriction 145 is preferably arranged in connection with or close to the ends 144 of the cell channels 140 in fluid connection with the output channel 120. Hence, the channel restrictions 145 in the cell channels 140 are preferably closer to the output channel 120 as compared to the input channel 110.

The exposing step S60 then comprises exposing bacterial cells captured in the test set or sets 130 of cell channels 140 to a test antimicrobial agent, steps S1 and S61 comprise exposing bacterial cells captured in a first set or sets 130 of cell channels 140 to the first antimicrobial agent or mixture, steps S2 and S62 comprise exposing bacterial cells captured in a second set or sets 130 of cell channels 140 to the second antimicrobial agent or mixture and exposing bacterial cells captured in a fourth set or sets 130 of cell channels 140 to the salt or salt mixture, step S3 comprises exposing bacterial cells captured in a third set or sets 130 of cell channels 140 to the third antimicrobial agent or mixture, and step S4 comprises exposing bacterial cells captured in a fifth set or sets 130 of cell channels 140 to a solvent, preferably a culture medium.

The microfluidic chip 10, see Figs. 18A-18C, 21, comprises a plurality of sets 130 of cell channels 140. The cell channels 140 have dimensions, such as width and height or diameter, sufficient to allow bacterial cells comprised in a biological sample to enter the cell channels 140 from an input channel 110. Hence, each set 130 of cell channels 140 comprises, as is shown in Fig. 21, a plurality of substantially parallel cell channels 140 interposed between and in fluid communication with an input channel 110 and an output channel 120. Hence, a first or input end 142 of the cell channels 140 is connected to the input channel 110 and a second or output end 144 of the cell channels 140 is connected to the output channel 120.

The loading of the biological sample comprising bacterial cells comprises entering bacterial cells into the cell channels 140 by flowing the biological sample through the input channel 110. The input channel 110 preferably has dimensions, e.g., width and height or dimeter, which are significantly larger than the diameter of the bacterial cells in the biological sample. The biological sample with the bacterial cells will flow through the input channel 110, typically from one of it ends towards its opposite end. The biological sample is also flowing through the cell channels 140 towards the output channel 120 and further out through at least one opening or output port 121 of the output channel 120. Bacterial cells present in the biological sample will thereby be transported through the input channel 110 and into cell channels 140. However, as the biological sample is flowing out through the second or output ends 144 and into the output channel 120 the bacterial cells will be captured by the channel restrictions 145 and thereby remain in the cell channels 140. Hence, the channel restrictions 145, also referred to as channel blocks herein, thereby prevent or at least restrict bacterial cells entering the cell channels 140 from flowing past the channel restrictions 145 and into the output channel 120. Each channel restriction 145 thereby defines a narrow passage in the portion of the cell channel 140 comprising the channel restriction 145 and where the narrow passage has dimensions, such as width and height or diameter, sufficient to allow the fluid or liquid part of the biological sample and any small cell debris present in the biological sample to pass through the narrow passage. However, any bacterial cells entering the cell channel 140 are too large to pass through this restricted narrow passage and thereby remain trapped in the cell channel 140.

The loading of the biological sample thereby results in bacterial cells present in the biological sample captured in the cell channels 140 of the plurality of sets 130. Some of the cell channels 140 in a set 130 may each comprise a single bacterial cell, some of the cell channels 140 in the set may each comprise multiple, i.e., at least two bacterial cells, whereas other cell channels 140 of the set 130 do not comprise any bacterial cells.

During loading, excess bacterial cells, or cells that are too large to fit in the cell channels 140, are washed out through a port 111 at the first end of the input channel 110. The liquid portion of the biological sample exits the set 130 of cell channels 140 both from the port 111 at the first end of the input channel 110 and the opening or output port 121 of the output channel 120.

The size selection capacity can be used to seed the cell channels 140 with bacterial cells that are separated from, for instance, blood cells that are significantly larger.

Once the bacterial cells have been captured in the cell channels 140, the bacterial cells could either directly be exposed to the antimicrobial agents or mixtures or the salt or salt mixture, or the bacterial cells are first allowed a period of time to grow and multiply in the cell channels 140 prior to exposing the bacterial cells in the cell channels 140 to the antimicrobial agents or mixtures or the salt or salt mixture or solvent, such as culture medium, in steps S1, S2, S3, S4, S60 and S61.

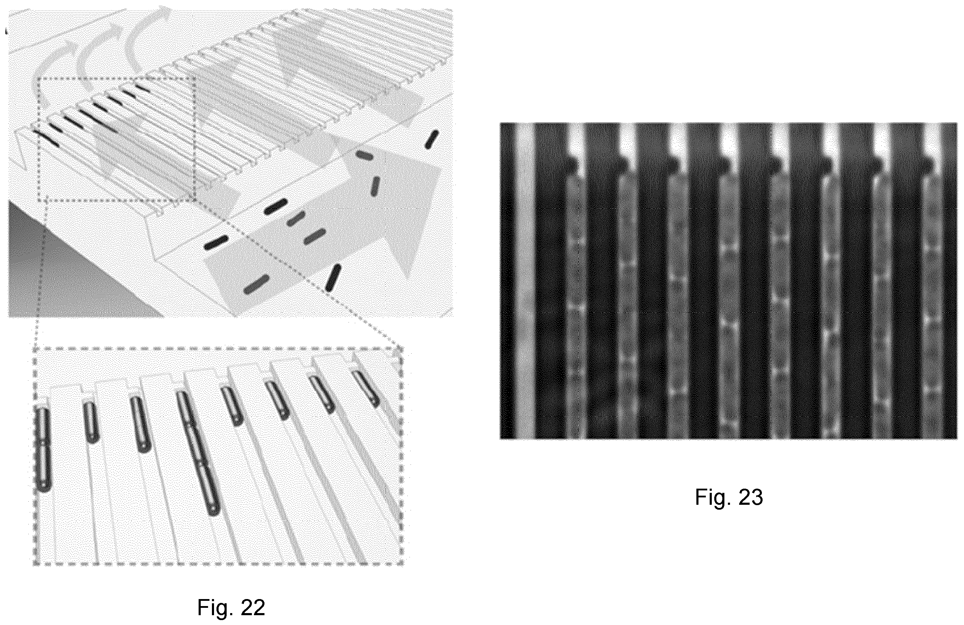

In the latter case, the bacterial cells captured in the cell channels 140 are allowed to grow along the length of the cell channels 140. The cell channels 140 are preferably dimensioned to allow the bacterial cells to grow in monolayer along the length of the cell channels 140. A monolayer culture simplifies visual characterization of individual bacterial cells as is further described herein. The embodiments are, however, not limited thereto. Thus, bacterial cells may alternatively grow in multilayer culture if the dimensions of the cell channels 140 are larger than the corresponding dimensions (cell diameter) of the bacterial cells. The bacterial cells in the cell channels 140 could preferably be seen as pearls on a string if the cell channels 140 are substantially one cell dimeter wide, see Figs. 22 and 23. If the cell channels 140 are wider the bacterial cells will form a 2D or even 3D layer in the cell channels 140.

Bacterial cells growing and mitigating past the first or input end 142 of the cell channels 140 will enter the input channel 110 and are thereby flushed away.

The microfluidic chip 10 of the microfluidic device 5 comprises a plurality of sets 130 of cell channels 140. This enables exposing any captured, and optionally cultured, bacterial cells to different antimicrobial agents or mixtures and salt or salt mixture at one or multiple different concentrations. For instance, a microfluidic chip 10 having 32 sets 130 of cell channels 140 could, as an example, be used for five different antimicrobial agents or mixtures at five concentrations and then using the remaining seven sets 130 of cell channels 140 to expose bacterial cells to one or more salts or salt mixtures at one or more different concentrations and control, such as merely exposing bacterial cells to culture medium.

In an embodiment, step S60 in Fig. 14 comprises exposing bacterial cells from the biological sample to multiple different concentrations of the test antimicrobial agent. Step S65 then comprises determining the MIC of the test antimicrobial agent based on the phenotype characteristic response of the bacterial cells to the multiple different concentrations of the test antimicrobial agent.

In this embodiment, at least two test sets 130 of cell channels 140, but preferably at least three test sets 130, more preferably at least four test sets 130 or at least five test sets 130 of cell channels are used to expose the captured bacterial cells to different concentrations of the test antimicrobial agent. The higher number of test sets 130 of cell channels 140, the higher number of different concentrations of the test antimicrobial agent can be tested and thereby the more accurate determination of a MIC of the test antimicrobial agent for the bacterial cells can be determined in step S65. In typical applications, two to eight, preferably three to seven, and more preferably four to six concentrations are sufficient for MIC determination and, hence, the microfluidic chip 10 comprises two to eight, preferably three to seven, and more preferably four to six test sets 130 of cell channels 140.

For instance, assume that the microfluidic chip 10 comprises five test sets 130 of cell channels 140 and that bacterial cells captured in each of these five test sets 130 of cell channels 140 is exposed to a respective concentration $X_1$ to $X_5$ of the test antimicrobial agent, wherein $X_1 < X_2 < X_3 < X_4 < X_5$. Bacterial cells are, in an example, viable and growing in the cell channels 140 of the test sets 130 of cell channels 140 with concentrations $X_1$ to $X_3$ but no cell growth is detected in the cell channels 140 of the test sets 130 of cell channels 140 with concentrations $X_4$ and $X_5$. In this illustrative example, the MIC of the antimicrobial agent could be determined in step S65 to be $X_4$ for the particular bacterial cells.

The MIC determination as performed in step S65, however, does not necessarily need to be based on the phenotype characteristic response of the bacterial cells to multiple different concentrations of the test antimicrobial agent. In fact, such MIC determination could be done by monitoring, preferably over time or at multiple scheduled time instances, the phenotype characteristic response of the bacterial cells to a single concentration of the test antimicrobial agent.

In such an embodiment, step S60 comprise exposing bacterial cells from the bacterial sample to a culture medium comprising at least one concentration, such as one, concentration of the test antimicrobial agent. In this embodiment, the method also comprises exposing bacterial cells from the biological sample to the culture medium lacking the test antimicrobial agent. In this embodiment, step S65 comprise determining the MIC of the test antimicrobial agent based on a normalized phenotype characteristic response obtained based on the phenotype characteristic response of the bacterial cells exposed to the at least one concentration of the test antimicrobial agent and a reference phenotype characteristic response of the bacterial cells exposed to the culture medium, preferably at multiple time instances.

In a particular embodiment, the MIC is determined in step S65 by calculating a value of normalized treatment growth rate mean (NTGRM), or of a parameter derived from or based on NTGRM. NTGRM represents an average growth rate of bacterial cells exposed to the test antimicrobial agent normalized by an average growth rate of bacterial cells exposed to the culture medium. An example of a parameter derived based on NTGRM is selected from the group consisting of standard error of the mean (SEM) in NTGRM calculated by diving the mean standard deviation in NTGRM to NTGRM for selected time instances and slope of NTRGRM calculated for selected time intervals.

In another particular embodiment, the MIC is determined in step S65 based on a value of a quotient between a mean growth rate of the bacterial cells exposed to the test antimicrobial agent and a mean growth rate of bacterial cells exposed to the culture medium. In another particular embodiment, the MIC is determined in step S65 based on a parameter based on, or equal to, 1 minus the above-mentioned value of the quotient.

The MIC determination in step S65 may be made based on the normalized phenotype characteristic response or parameter above and antimicrobial MIC decision support information representing a relationship between normalized phenotype responses or parameter and MIC values, see Fig. 17.

This MIC decision support information may be determined by a method comprising the following steps performed for each bacterial strain of multiple bacterial strains having a respective MIC value with regard to an antimicrobial agent.

Exposing bacterial cells to a culture medium comprising at least one concentration of the antimicrobial agent and exposing bacterial cells to the culture medium lacking the antimicrobial agent. Monitoring bacterial cells at multiple time instances and determining a normalized phenotype characteristic response based on the monitoring of bacterial cells at the multiple time instances. The normalized phenotype characteristic response is obtained based on a phenotype characteristic response of the bacterial cells exposed to the culture medium comprising the at least one concentration of the antimicrobial agent and a phenotype characteristic response of bacterial cells exposed to the culture medium. The method then comprises generating the antimicrobial MIC decision support information based on the normalized phenotype characteristic responses for the multiple bacterial strains and the MIC values of the multiple microbial strains.

In a particular embodiment, the antimicrobial MIC decision support information represents a relationship between the normalized phenotype characteristic responses for the multiple bacterial strains and the MIC values of the multiple bacterial strains. For instance, the antimicrobial MIC decision support information could represent a relationship $MIC = f(x)$, wherein $x$ represents a normalized phenotype characteristic response and $f()$ is a function having parameters determined based on the normalized phenotype characteristic responses for the bacterial microbial strains and the MIC values of the multiple bacterial strains, see Fig. 17.

Exposing the bacterial cells in the cell channels to the antimicrobial agents or mixtures in steps S1, S2, S3, S60, S61 and to the salt or salt mixture in step S2 preferably comprises flowing a culture medium comprising the respective antimicrobial agent or mixture or the salt or salt mixture dissolved or dispersed in the culture medium into the cell channels 140. Hence, culture medium comprising a dissolved or dispersed antimicrobial agent or mixture or salt or salt mixture at a defined concentration is preferably directed into the input channel 110 of a set 130 of cell channels 140. The culture medium with the dissolved or dispersed antimicrobial agent or mixture or salt or salt mixture thereby flows into and through the cell channels 140 and out into the output channel 120 and further out through the at least one opening 121 of the output channel 120. This flow of culture medium with dissolved or dispersed antimicrobial agent or mixture or salt or salt mixture is preferably performed for a period of time to expose the bacterial cells to the antimicrobial agent or mixture or salt or salt mixture at the defined concentration while monitoring the phenotype characteristic response of the bacterial cells to the antimicrobial agent or mixture or salt or salt mixture. Each set 130 of cell channels 140 preferably comprise a respective source of culture medium with dissolved or dispersed antimicrobial agent with no cross-talk between sets 130 of channels 140. It is, though, possible that bacterial cells in cell channels 140 of two or more sets 130 of cell channels 140 are exposed to the same concentration of the same antimicrobial agent or mixture or salt or salt mixture and may then share a single common source of culture medium with dissolved or dispersed antimicrobial agent or mixture or salt or salt mixture.

At least some of the method steps in Figs. 2 to 15 could be computer-implemented steps executed by a processor. As illustrative, but non-limiting, examples step S5 in Fig. 2, steps S10-S12 in Fig. 3, steps S20-S24 in Fig. 4, steps S30-S32 in Fig. 7, steps S40-S42 in Fig. 8, steps S50-S52 in Fig. 9, steps S62-S64 in Fig. 14 and/or step S65 in Fig. 15 could be such computer-implemented steps executed by a processor.

The methods of Figs. 2 and 14 preferably also comprise monitoring the bacterial cells to determine phenotype characteristic response of the bacterial cells to the antimicrobial agents.

Various types of phenotype characteristic responses, also referred to as phenotypic (characteristic) responses herein, could be monitored and analyzed including, but not limited to, growth rate, shape, size, form of growth rate curve defining growth rate over time, form of length curve defining cell length over time, form of area curve defining cell area over time, color, optical density, electrical conductivity, heat production, surface antigen composition as observed by affinity reagents, absorption spectra, and a mixture of at least two such phenotype characteristics.

Growth rate is a phenotype characteristic or trait that can advantageously be used to determine the response of the bacterial cells to the antimicrobial agents or mixtures or to the salt or salt mixtures. Growth rate can be determined, for instance, by monitoring the number of bacterial cells in each cell channel 140 as the number will increase over time for growing cells. Alternatively, or in addition, grow rate can be determined by monitoring the length of the portion of a cell channel 140 occupied by bacterial cells, see Figs. 22 and 23. This length will increase over time for growing cells but remain the same for non-viable and non-growing cells. Alternatively, or in addition, grow rate can be determined by monitoring the area or length of cells segmented in images of the cell channels 140

The growth rate over time typically may vary depending on the presence of any antimicrobial agent or mixture or salt or salt mixture in the culture medium and/or the concentration of the antimicrobial agent or mixture or salt or salt mixture. In some instances, the bacterial cells grow exponentially, whereas in other instances the bacterial cells grow in more periodic ways. Accordingly, the shape or form of the growth rate curve can be used to determine the phenotypic characteristic response of the bacterial cells to the antimicrobial agents or mixtures or to the salt or salt mixture.

Other phenotype characteristics that may vary for the cells in the absence versus presence of the antimicrobial agents or mixtures or salt or salt mixture include the shape, size, color and optical density. Optical density, color or other spectral properties may differ depending on contents of the bacterial cells, shape of the bacterial cells, etc. Thus, optical properties can be used to determine the responses of the bacterial cells to the antimicrobial agents or mixtures or salt or salt mixture.

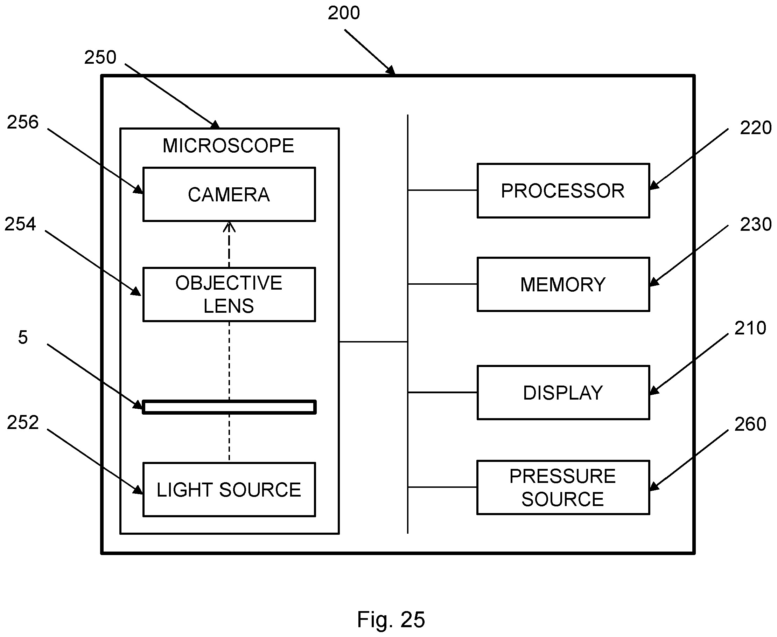

These above exemplified phenotype characteristic responses are preferably determined optically by a camera 256 or other instrument capable of taking pictures or video of the portion of the microfluidic chip 10 comprising the plurality of sets 130 of cell channels 140, see Fig. 25. The microfluidic chip 10 is then preferably made of an optically transparent material to enable taking pictures or video of the bacterial cells in the cell channels 140.

A typical, but non-limiting, example of material for the microfluidic chip 10 is liquid silicone rubber (LSR).

The bacterial cells in the cell channels 140 could be monitored either more or less continuously or at multiple time instances as the culture medium with dissolved or dispersed antimicrobial agents are flowing through the cell channels 140. For instance, one or more video cameras could be used for monitoring the phenotype characteristic responses of the bacterial cells to the antimicrobial agents or mixtures or salt or salt mixture or one or more cameras could be used to take pictures of the plurality of sets 130 of cell channels 140 at selected time instances.

In a particular embodiment, pictures are taken of the bacterial cells in the cell channels 140 using a microscopy, such as a phase contrast microscope, connected to a camera, such as charge-coupled device (CCD) and complementary metal-oxide semiconductor (CMOS) camera, or a confocal scanning system for fluorescence, Raman imaging, Coherent Anti-stokes Raman Scattering (CARS), Stimulated Raman Scattering (SRS) and similar chemically sensitive techniques that gives spectral changes for dead and live cells. This includes measurements in one or several wavelengths with or without contrast enhancing additions to the growth media, such as chemically specific probes and dyes.

Conductivity and heat production will depend on the chemical composition and metabolic state of the bacterial cells and can therefore constitute the basis for determining the cellular responses to the antimicrobial agents. Conductivity and/or heat production could be measured by electrodes or sensors of the instrument arranged in or in connection with the cell channels 140.

The present invention is useful when analyzing a biological sample containing bacteria and taken from a subject with the purpose of selecting antimicrobial agent to treat the bacterial infection in the subject. For instance, assume that a subject is suffering from UTI and a urine sample is taken from the subject as a biological sample. Bacterial cells from the urine sample are then exposed to the antibiotic ciprofloxacin (CIP) as test antimicrobial agent in step S60. Assume that the MIC of CIP is determined in step S63 to be 1 $\mu$g/mL. It is not possible to conclude from this determined MIC value *per se* whether the subject could be effectively treated by CIP against the UTI. UTIs may be caused by various different bacterial cells including *Escherichia coli* (gram negative bacteria) and *Enterococcus faecalis* (gram positive bacteria). Assume that the clinical breakpoint of CIP is 0.25 $\mu$g/mL for UTI-causing gram negative bacteria, such as *E. coli*, but 4 $\mu$g/mL for UTI-causing gram positive bacteria, such as *E. faecalis.* In this illustrative example, the MIC determined in step S63 is higher than the clinical breakpoint of CIP for UTI-causing gram negative bacteria but lower than the clinical breakpoint of CIP for UTI-causing gram positive bacteria. This means that the UTI infection could be effectively treated by CIP if caused by, for instance, *E. faecalis* but not if caused by, for instance, *E. coli.*

The method as disclosed in Fig. 14 enables determination of correct CBP of, in this example, CIP based on phenotype characteristic response(s) of the UTI-causing bacterial cells to at least first and second antimicrobial agents or mixtures in step S1 and S2 of Fig. 2. A classification of the bacterial cells is then done in step S5 of Fig. 2 and used to determine the CBP in step S62 of Fig. 14. The method of Fig. 14 can thereby be used to determine whether the UTI-causing bacterial cells are susceptible (*E. faecalis*) or not (*E. coli*) to CIP based on a comparison of the MIC determined in step S63 and the CBP determined in step S62.

Illustrative, but non-limiting, examples of antimicrobial agent that can be used according to the invention include glycopeptides, vancomycin, teicoplanin, cephalosporings, such as cefepime, β-lactamase inhibitor pencillins, such as piperacillin, tazobactam, ticarcillin, clavulanate, and carbapenems, such as imipenem, cilastatin, meropenem, ertapenem.

Non-limiting, but illustrative, examples of salts that could be used according to the embodiments include sodium chloride (NaCl) and potassium chloride (KCl).

With reference to Figs. 18A and 18B, the microfluidic chip 10 is preferably attached and bonded to a chip carrier 20 prior to arranging the microfluidic chip 10 and chip carrier 20 in a substrate 1, see Fig. 19. In an embodiment, the microfluidic chip 10 is attached to the chip carrier 20, for instance by bonding the microfluidic chip 10 onto the chip carrier 20 as shown in Fig. 18B. The chip carrier 20 with the attached microfluidic chip 10 is then inserted into a matching chip chamber or well 2 in the substrate 1 as indicated in Fig. 19. The chip carrier 20 is preferably attached to the substrate 1, such as by welding, preferably laser welding, the chip carrier 20 to the substrate 1. Fig. 20 illustrates an embodiment of the microfluidic device 5 ready for conducting the method shown in Fig. 2 or 14. A lid 3 has been attached to the substrate 1 and the lid 3 preferably comprises an opening 4 aligned with at least the portion of the microfluidic chip 10 comprising the plurality of sets 130 of cell channels 140. Accordingly, these cell channels 140 can be monitored and imaged during operation of the microfluidic device 5 through the opening 4.

As an illustrative example, the chip carrier 20 may be made of polystyrene, such as optically transparent polystyrene.

In an embodiment, the chip carrier 20 comprises two matrices or arrays of entrance holes 31 to 38 providing access to the microfluidic chip 10 via channels in the chip carrier 20, see Figs. 18C. Each matrix preferably comprises four columns 21 to 28 of holes 31 to 38 and N rows of holes 31 to 38. The chip carrier 20 preferably also comprises a central window 29 aligned with the plurality of sets 130 of cell channels 140.

The holes 31, 32 in the first columns 21, 22 closest to a central window 29 in the chip carrier 20 are preferably connected to a reservoir 244 (Fig. 21) comprising culture medium with at least one surfactant.

The culture medium with the at least one surfactant is preferably employed to wet the channels 110, 120, 140 in the microfluidic chip 10 to thereby facilitate subsequent filling steps of adding the biological sample and exposing any captured bacterial cells to antimicrobial agents or mixtures and salt or salt mixture. Each hole 31, 32 in the first columns 21, 23 is connected to an opening or output port 121 of a respective output channel 120 of a set 130 of cell channels 140. The holes 33, 34 in the second columns 23, 24 are preferably connected to a sample reservoir 241 (Fig. 21) comprising the biological sample. Each hole 33, 34 in the second columns 23, 24 is connected to a respective input channel 110 of a set 130 of cell channels 140. The holes 35, 36 in the third columns 25, 26 are preferably connected to a waste reservoir 243 (Fig. 21) designed to house any excessive fluid or liquid from the plurality of sets 130 of cell channels 140. The holes 35, 36 in the third columns 25, 26 are connected to a respective port 111 of the first end of the input channels 110 of the sets 130 of cell channels 140. The holes 37, 38 in the outermost, fourth columns 27, 28 are each connected to a respective agent reservoir 242 (Fig. 21) comprising an antimicrobial agent or mixture or salt or salt mixture at a defined concentration dissolved or dispersed in culture medium or only culture medium. Each hole 37, 38 in the fourth columns 27, 28 is connected to a respective input channel 110 of a set 130 of cell channels 140.

Prior to loading the biological sample into the microfluidic chip 10, the channels 110, 120, 140 of the microfluidic chip 10 are preferably wetted to remove any air captured in these channels 110, 120, 140. The wetting of the microfluidic chip 10 is performed with the culture medium comprising at least one surfactant contained in a reservoir 244. Hence, a pressure is applied at a pressure port, which is in fluid connection with the reservoir 244. The culture medium comprising surfactant is thereby pushed from the reservoir 244 into the first holes 31, 32 of the chip carrier 20. The culture medium with surfactant thereby enters the sets 130 of cell channels 140 in the microfluidic chip 10 through the openings or output port 121 of the output channels 120, flows through the cell channels 140 into the input channels 110 and out through the ports 111 at the first ends of the input channels 130. Excess culture medium with surfactant then flows into the holes 35, 37 in the third columns 25, 26 and further into the waste reservoir 243. This initial wetting of the microfluidic chip 10 removes any air trapped in the microfluidic chip 10, i.e., in the set 130 of cell channels 140 and the input and output channels 110, 120. Additionally, the surfactant dissolved or dispersed in the culture medium coats the surfaces of the cell channels 140 and input and output channels 110, 120 to thereby reduce the flow resistance for the biological sample and the culture medium with dissolved or dispersed antimicrobial agents.

Once the wetting of the microfluidic chip 10 is finished, the biological sample is pushed into microfluidic chip 10 to therein capture any bacterial cells present in the biological sample in the cell channels 140. A pressure is applied at a pressure port in fluid connection with the sample reservoir 241. The biological sample in the sample reservoir 241 is thereby pushed into the holes 33, 34 in the second columns 23, 24 in the chip carrier 20. The biological sample is further pushed into ports 112 at the second ends of the input channels 110 and then into the cell channels 140. The biological sample is further flown out through the second or output ends 144 of the cell channels 140, into the output channels 120 and out through the openings or output ports 121 and then leaves the microfluidic chip 10 and chip carrier 20 via the holes 31, 32 in the first columns 21, 22 of the chip carrier 20. Bacterial cells present in the biological sample will be trapped in the cell channels 140 by the cell restrictions 145 provided therein. Excess biological sample is allowed to flow out through the ports 111 at the first ends of the input channels 110 and to the waste reservoirs 243 connected to the holes 35, 36 in the third columns 25, 26 of the chip carrier 20.

At this point, bacterial cells present in the biological sample have been captured in the cell channels 140 in the substrate 10 and can now be exposed to antimicrobial agents or mixtures or a salt or salt mixture dissolved or dispersed in the culture medium in the loaded agent reservoirs 242. Accordingly, pressure is applied at pressure ports that are in fluid connection with the agent reservoirs 242. The applied pressure presses the culture medium with dissolved or dispersed antimicrobial agent or mixture or salt or salt mixture, or no dissolved agent or salt, out through the agent reservoirs 242 and into the respective holes 37, 38 in the fourth columns 27, 28 of the chip carrier 20. The culture medium with dissolved or dispersed antimicrobial agent or mixture or salt or salt mixture is further pushed into the ports 111 at the first ends of the input channels 110 and flows into the cell channels 140 and out into the output channels 120 and the openings 121. This flow of culture medium means that bacterial cells captured in cell channels 140 in one set 130 of cell channels 140 are exposed to a defined concentration of an antimicrobial agent or mixture or a salt or salt mixture, whereas bacterial cells captured in cell channels 140 in another set 130 of cell channels 140 may be exposed to another defined concentration of the antimicrobial agent or mixture or salt or salt mixture, to a defined concentration of another antimicrobial agent or mixture or another salt or salt mixture, or merely to culture medium. By having multiple sets 130 of cell channels 140, such as $2 \times N$, or 32, as shown in the figures, multiple different antimicrobial agents or mixtures and various concentrations of these multiple different antimicrobial agents or mixtures can be tested for one particular biological sample and still allowing internal controls in one or more sets 130 of cell channels 140. Excess culture medium with any dissolved or dispersed antimicrobial agent is flown from the openings or output ports 121 into the holes 31, 32 of the first columns 21, 21 and then out into a waste reservoir 243.

The microfluidic chip 10 preferably comprises two matrices or arrays, each having N rows of holes 31 to 38 as mentioned above. As a consequence, the microfluidic chip 10 preferably comprises $2 \times N$ sets 130 of cell channels 140. For instance, assume that N is 16, then the microfluidic chip 10 comprises 32 sets 130 of cell channels 140. In an example, the microfluidic chip 10 and microfluidic device 5 could be used to test five different antimicrobial agents or mixtures at five different concentrations and then using the remaining seven sets 130 of cell channels 140 to expose bacterial cells to one or more salts or salt mixtures at one or more different concentrations and control, such as merely exposing bacterial cells to culture medium. In this illustrative example, up to five different MIC values could be determined for the five different antimicrobial agents in step S65 and up to five CBPs can be determines in step S64 for these five different antimicrobial agents.

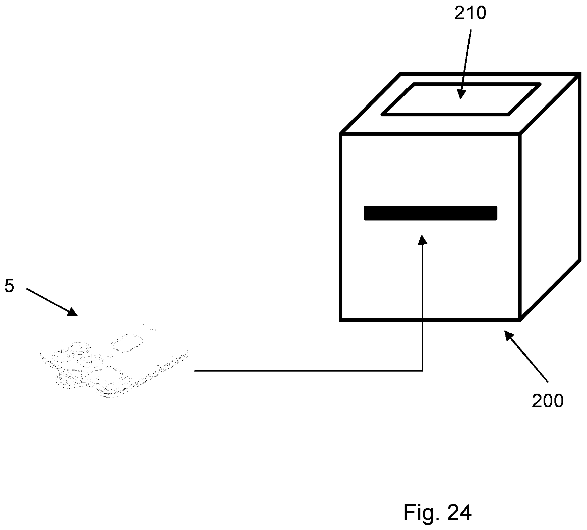

The methods of the present invention are preferably performed by an analyzer 200 as shown in Figs. 24 and 25. In such an embodiment, the analyzer 200 is configured to receive a microfluidic device 5 preloaded with culture medium, surfactant, and antimicrobial agents or mixtures or a salt or salt mixture, see Fig. 21. The biological sample is preferably also added to the sample reservoir 241 in the microfluidic device 5 prior to inserting the microfluidic device 5 in a receptacle in the analyzer 200 as schematically indicated in Fig. 24.

The analyzer 200 is then configured to perform the methods of the present invention to preferably expose bacterial cells from a biological sample to a test antimicrobial agent in the microfluidic device 5, to expose bacterial cells from the biological sample to a first antimicrobial agent or mixture in the microfluidic device 5, expose bacterial cells from the biological sample to (i) a second antimicrobial agent or mixture in the microfluidic device 5 and/or (ii) to a salt or salt mixture in the microfluidic device 5, to classify any bacteria presented in the loaded biological sample and preferably also to determine the MIC of the bacteria. The result of the operations of the analyzer 200 can then be presented to a user on a display 210 of or connected (wired or wirelessly connected) to the analyzer 200.

Fig. 25 is a schematic block diagram of an analyzer 200 according to an embodiment. The analyzer 200 comprises a processor 220 and a memory 230 interconnected to each other to enable normal software execution. In such a case, the memory 230 comprises instructions executable by the processor 220 to cause the processor 220 to perform the methods of the present invention. The analyzer 200 also comprises a display 210, a microscope 250, and a pressure source 260.

The term processor 220 should be interpreted in a general sense as any circuitry, system or device capable of executing program code or computer program instructions to perform a particular processing, determining or computing task. The processors 220 is, thus, configured to perform, when executing the computer program, well-defined processing tasks such as those described herein. As an example, the processor 220 could be implemented using any combination of one or more of a suitable central processing unit (CPU), multiprocessor, microcontroller, digital signal processor (DSP), etc., capable of executing software instructions stored in the memory 230. The processor 220 may further be provided as at least one application specific integrated circuit (ASIC), or field programmable gate array (FPGA).

The memory 230 may also comprise persistent storage, which, for example, can be any single one or combination of magnetic memory, optical memory, solid state memory or even remotely mounted memory.

The processor 220 is, as previously mentioned herein, configured to control the pressure source 260 to control the flow of fluids in the microfluidic device 5, see Fig. 21. The pressure source 260 preferably comprises a pump and a motor configured to drive the pump. The processor 220 is preferably also configured to control the microscope 250 of or connected to the analyzer 250. The microscope 250 preferably comprises a light source 252 configured to illuminate the sets 130 of cells channels 140 in the microfluidic device 5 and a light detector 256, represented by a camera 256 in Fig. 25. This camera 256 is then configured to detect light having passed through or reflected from the sets 130 of cell channels 140 to determine one or more images or video frames of the sets 130 of cell channels 140, such as shown in Fig. 23. The analyzer 200 could comprise a single camera 256 sized to monitor all the sets 130 of cell channels 140 in the microfluidic device 5 or multiple cameras 256 that can be operated serially or at least partly in parallel to monitor the sets 130 of cell channels 140. Alternatively, the camera 256 is movable relative to the sets 130 of cell channels 140 in the microfluidic device 5 to sequentially monitor the different sets 130 of cell channels 140.

The microscope 250 may optionally comprise one or more objective lenses 254 arranged in a light path between the light source 252 and the camera 256, such as upstream and/or downstream of the microfluidic device 5.

The operation of the microscope 250 is then preferably controlled by the processor 220 of the analyzer 200.

The processor 220 is further configured to process the images or video data from the camera 256 in order to determine the phenotypic response of the bacterial cells to various antimicrobial agents or mixtures, and the salt(s) or salt mixture(s).

An aspect of the invention therefore relates to an analyzer 200 comprising a pressure source 260 configured to apply pressure to a microfluidic device 5, a processor 220 and a memory 230 comprising instructions executable by the processor 220 to cause the processor 220 to control the pressure source 260 to expose bacterial cells from a biological sample to a first antimicrobial agent or mixture in the microfluidic device 5 and to control the pressure source 260 to expose bacterial cells from the biological sample to (i) a second antimicrobial agent or mixture in the microfluidic device 5 and/or (ii) to a salt or salt mixture in the microfluidic device 5. The processor 220 is also caused to classify the bacterial cells from the biological sample at least based on (i) a phenotype characteristic response of the bacterial cells to the first antimicrobial agent or mixture and (iia) a phenotype characteristic response of the bacterial cells to the second antimicrobial agent or mixture and/or (iib) a phenotype characteristic response of the bacterial cells to the salt or salt mixture.

In an embodiment, the processor 220 is caused to control the pressure source 260 to apply pressure to a microfluidic device 5, comprising multiple sets 130 of cell channels 140 configured to capture the bacterial cells, to flow the first antimicrobial agent or mixture onto bacterial cells captured in at least one first set 130 of cell channels 140 of the multiple sets 130 of cell channels 140 and flow the second antimicrobial agent or mixture onto bacterial cells captured in at least one second set 130 of cell channels 140 of the multiple sets 130 of cell channels 140 and/or flow the salt or salt mixture onto bacterial cells captured in at least one fourth set 130 of cell channels 140 of the multiple sets 130 of cell channels 140.

Another aspect of the invention relates to an analyzer 200 comprising a pressure source 260 configured to apply pressure to the microfluidic device 5, a processor 220 and a memory 230 comprising instructions executable by the processor 220 to cause the processor 220 to control the pressure source 260 to expose bacterial cells from a biological sample to a test antimicrobial agent in the microfluidic device 5, to control the pressure source 260 to expose bacterial cells from the biological sample to a first antimicrobial agent or mixture in the microfluidic device 5, and to control the pressure source 260 to expose bacterial cells from the biological sample to (i) a second antimicrobial agent or mixture in the microfluidic device 5 and/or (ii) to a salt or salt mixture in the microfluidic device 5. The processor 220 is also caused to classify the bacterial cells at least based on (i) a phenotype characteristic response of the bacterial cells to the first antimicrobial agent or mixture and (iia) a phenotype characteristic response of the bacterial cells to the second antimicrobial agent or mixture and/or (iib) a phenotype characteristic response of the bacterial cells to the salt or salt mixture. The processor 220 is further caused to determine a clinical breakpoint for the test antimicrobial agent based on a result of classifying the bacterial cells and determine a minimum inhibitory concentration of the test antimicrobial agent based on a phenotype characteristic response of the bacterial cells to the test antimicrobial agent. The processor 220 is additionally caused to compare the minimum inhibitory concentration with the clinical breakpoint.

In an embodiment, the processor 220 is caused to control a pressure source 260 to apply pressure to a microfluidic device 5, comprising multiple sets 130 of cell channels 140 configured to capture the bacterial cells, to flow the test antimicrobial agent to at least one test set 130 of cell channels 140 of the multiple sets 130 of cell channels 140, flow the first antimicrobial agent or mixture onto bacterial cells captured in at least one first set 130 of cell channels 140 of the multiple sets 130 of cell channels 140 and flow the second antimicrobial agent or mixture onto bacterial cells captured in at least one second set 130 of cell channels 140 of the multiple sets 130 of cell channels 140 and/or flow the salt or salt mixture onto bacterial cells captured in at least one fourth set 130 of cell channels 140 of the multiple sets 130 of cell channels 140.

The processor 220 is preferably also caused to perform the various method steps disclosed herein in connection with Figs. 2 to 15 by executing instructions stored in the memory 230.

In an embodiment, the analyzer 200 comprises the microfluidic device 5.

In an embodiment, the analyzer 200 comprises a microfluidic device 5, a pressure source 260 configured to apply pressure to the microfluidic device 5, a camera 256, a processor 220 and a memory 230. The memory 230 comprises instructions executable by the processor 220 to cause the processor 220 to:

control the pressure source 260 to expose bacterial cells from a biological sample to an antimicrobial agent or mixture in the microfluidic device 5;

control the camera 256 to take images of bacterial cells from the biological sample captured in the microfluidic device 5 at multiple time instances while the bacterial cells are not exposed to any antimicrobial agent or mixture;

determine a morphology classification of the bacterial cells based on the images; and

classify the bacterial cells from the biological sample based on the morphology classification and a phenotype characteristic response of the bacterial cells to the antimicrobial agent or mixture.

In an embodiment, the memory 230 comprises instructions executable by the processor 220 to cause the processor 220 to:

control the pressure source 260 to expose bacterial cells from the biological sample to a test antimicrobial agent;

determine a clinical breakpoint for the test antimicrobial agent based on a result of the classification of the bacterial cells;

determine a minimum inhibitory concentration of the test antimicrobial agent based on a phenotype characteristic response of the bacterial cells to the test antimicrobial agent; and

compare the minimum inhibitory concentration with the clinical breakpoint.

Various embodiments of the invention are provided by the following items.

Item 1: A method for classifying bacteria, the method comprising:

exposing (S1) bacterial cells from a biological sample to a first antimicrobial agent or mixture;

exposing (S2) bacterial cells from the biological sample to (i) a second antimicrobial agent or mixture and/or (ii) a salt or salt mixture; and

classifying (S5) the bacterial cells from the biological sample at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture.

Item 2: The method according to Item 1, wherein classifying (S5) the bacterial cells comprises:

classifying (S5) the bacterial cells at least based on the phenotype characteristic response of the bacterial cells to the first antimicrobial agent or mixture and the phenotype characteristic response of the bacterial cells to the second antimicrobial agent or mixture if valid data of the phenotype characteristic response of the bacterial cells to the second antimicrobial agent or mixture is available; and

classifying (S5) the bacterial cells at least based on the phenotype characteristic response of the bacterial cells to the first antimicrobial agent or mixture and the phenotype characteristic response of the bacterial cells to the salt or salt mixture if no valid data of the phenotype characteristic response of the bacterial cells to the second antimicrobial agent or mixture is available.

Item 3: The method according to Item 1 or 2, wherein classifying (S5) the bacterial cells comprises classifying (S12) the bacterial cells at least based on (i) the phenotype characteristic response of the bacterial cells to the first antimicrobial agent or mixture, and (iia) the phenotype characteristic response of the bacterial cells to the second antimicrobial agent or mixture and/or (iib) the phenotype characteristic response of the bacterial cells to the salt or salt mixture if the bacterial cells have a predefined gram classification, preferably gram positive.

Item 4: The method according to Item 3, wherein classifying (S12) the bacterial cells comprises classifying (S51, S52) the bacterial cells as *Staphylococcus* or *Enterococcus.*

Item 5: The method according to Item 3 or 4, further comprising:

exposing (S3) bacterial cells from the biological sample to a third antimicrobial agent or mixture, wherein

classifying (S12) the bacterial cells comprises classifying (S12) the bacterial cells based on at least two of the phenotype characteristic response of the bacterial cells to the first antimicrobial agent or mixture, the phenotype characteristic response of the bacterial cells to the second antimicrobial agent or mixture, the phenotype characteristic response of the bacterial cells to the salt or salt mixture, and a phenotype characteristic response of the bacterial cells to the third antimicrobial agent or mixture.

Item 6: The method according to Item 5, wherein classifying (S12) the bacterial cells comprises:

classifying (S12) the bacterial cells based on the phenotype characteristic responses of the bacterial cells to at least two of the first, second and third antimicrobial agents or mixtures if valid data of the phenotype characteristic responses of the bacterial cells to the at least two of the first, second and third antimicrobial agents or mixtures are available; and

classifying (S12) the bacterial cells based on the phenotype characteristic response of the bacterial cells to the salt or salt mixture and the phenotype characteristic response of the bacterial cells to one of the first, second and third antimicrobial agents or mixtures if valid data of the phenotype characteristic response of the bacterial cells to merely one of the first, second and third antimicrobial agents or mixtures is available.

Item 7: The method according to any one of Items 1 to 6, wherein classifying (S5) the bacterial cells comprises determining (S10) a gram classification for the bacterial cells at least based on the phenotype characteristic response of the bacterial cells to an antimicrobial agent or mixture, at least one morphology characteristic of the bacterial cells from the biological sample, or the phenotype characteristic response of the bacterial cells to the salt or salt mixture.

Item 8: The method according to Item 7, wherein the antimicrobial agent or mixture is the first antimicrobial agent or mixture.

Item 9: The method according to Item 7 or 8, wherein determining (S10) the gram classification comprises determining (S22) the gram classification for the bacterial cells at least based on the phenotype characteristic response of the bacterial cells to the antimicrobial agent or mixture and the at least one morphology characteristic of the bacterial cells from the biological sample if valid data of the at least one morphology characteristic of the bacterial cells and valid data of the phenotype characteristic response of the bacterial cells to the antimicrobial agent or mixture are available.

Item 10: The method according to any one of Items 7 to 9, wherein determining (S10) the gram classification comprises determining (S23) the gram classification for the bacterial cells at least based on the at least one morphology characteristic of the bacterial cells from the biological sample if valid data of the at least one morphology characteristic of the bacterial cells is available but no valid data of the phenotype characteristic response of the bacterial cells to the antimicrobial agent or mixture is available.

Item 11: The method according to any one of Items 7 to 10, wherein determining (S10) the gram classification comprises determining (S25) the gram classification for the bacterial cells at least based on the phenotype characteristic response of the bacterial cells to the antimicrobial agent or mixture if valid data of the phenotype characteristic response of the bacterial cells to the antimicrobial agent or mixture is available but no valid data of the at least one morphology characteristic of the bacterial cells is available.

Item 12: The method according to any one of Items 7 to 11, wherein determining (S10) the gram classification comprises determining (S10) the gram classification for the bacterial cells at least based on the phenotype characteristic response of the bacterial cells to the salt or salt mixture if no valid data of the at least one morphology characteristic of the bacterial cells and no valid data of the phenotype characteristic response of the bacterial cells to the antimicrobial agent or mixture are available.

Item 13: The method according to any one of Items 7 to 12, wherein classifying (S5) the bacterial cells comprises classifying (S50) the bacterial cells as Enterobacterales if the determined gram classification is gram negative.

Item 14: A method for analyzing bacteria, the method comprises:

exposing (S60) bacterial cells from a biological sample to a test antimicrobial agent; classifying (S61) the bacterial cells according to any one of Items 1 to 13;
determining (S62) a clinical breakpoint for the test antimicrobial agent based on a result of classifying the bacterial cells;
determining (S63) a minimum inhibitory concentration of the test antimicrobial agent based on a phenotype characteristic response of the bacterial cells to the test antimicrobial agent; and
comparing (S64) the minimum inhibitory concentration with the clinical breakpoint.

Item 15: The method of Item 14, further comprising determining (S65) whether the bacterial cells are susceptible to the test antimicrobial agent based on the comparison between the minimum inhibitory concentration and the clinical breakpoint.

Item 16: The method of Item 14 or 15, wherein

exposing (S60) the bacterial cells to the test antimicrobial agent comprises exposing (S60) bacterial cells captured in a test set or sets (130) of cell channels (140) having a respective channel restriction (145) configured to restrict bacterial cells entering the cell channels (140) from leaving the cell channels (140) to the test antimicrobial agent;

exposing (S1) the bacterial cells to the first antimicrobial agent or mixture comprises exposing (S1) bacterial cells captured in a first set or sets (130) of cell channels (140) having a respective channel restriction (145) configured to restrict bacterial cells entering the cell channels (140) from leaving the cell channels (140) to the first antimicrobial agent or mixture; and

exposing (S2) the bacterial cells to the second antimicrobial agent or mixture comprises exposing (S2) bacterial cells captured in a second set or sets (130) of cell channels (140) having a respective channel restriction (145) configured to restrict bacterial cells entering the cell channels (140) from leaving the cell channels (140) to the second antimicrobial agent or mixture, and exposing (S2) the bacterial cells to the salt or salt mixture comprises exposing (S2) bacterial cells captured in a third set or sets (130) of cell channels (140) having a respective channel restriction (145) configured to restrict bacterial cells entering the cell channels (140) from leaving the cell channels (140) to the salt or salt mixture.

Item 17: The method of Item 16, wherein the test set or sets (130) of cell channels (140), the first set or sets (130) of cell channels (140), the second set or sets (130) of cell channels (140) and the third set or sets (130) of cell channels (140) are formed as separate compartments in a microfluidic chip (10).

Item 18: The method according to any one of the Items 1 to 17, wherein the phenotype characteristic response(s) is selected from the group consisting of growth rate, degree of nucleoid compaction, degree of metabolic activity and degree of membrane integrity.

Item 19: An analyzer (200) comprising:

a pressure source (260) configured to apply pressure to a microfluidic device (5), a processor (220); and a memory (230) comprising instructions executable by the processor (220) to cause the processor (230) to:

control the pressure source (260) to expose bacterial cells from a biological sample to a first antimicrobial agent or mixture in the microfluidic device (5);
control the pressure source (260) to expose bacterial cells from the biological sample to (i) a second antimicrobial agent or mixture and/or (ii) a salt or salt mixture in the microfluidic device (5); and

classify the bacterial cells from the biological sample at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture.

Item 20: An analyzer (200) comprising:

a pressure source (260) configured to apply pressure to a microfluidic device (5), a processor (220); and a memory (230) comprising instructions executable by the processor (220) to cause the processor (230) to:

control the pressure source (260) to expose bacterial cells from a biological sample to a test antimicrobial agent in the microfluidic device (5);
control the pressure source (260) to expose bacterial cells from the biological sample to a first antimicrobial agent or mixture in the microfluidic device (5);
control the pressure source (260) to expose bacterial cells from the biological sample to (i) a second antimicrobial agent or mixture and/or (ii) a salt or salt mixture in the microfluidic device (5);
classify the bacterial cells at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture;
determine a clinical breakpoint for the test antimicrobial agent based on a result of classifying the bacterial cells;
determine a minimum inhibitory concentration of the test antimicrobial agent based on a phenotype characteristic response of the bacterial cells to the test antimicrobial agent; and
compare the minimum inhibitory concentration with the clinical breakpoint.

Item 21: The analyzer according to Item 19 or 20, further comprising the microfluidic device (5).

EXAMPLES

EXAMPLE 1

The present Example investigates the feasibility of the analyzing method in Fig. 14 to determine whether bacterial cells from a urine sample from a patient suffering from UTI are susceptible or not to ciprofloxacin (CIP).

The experiments in the current Example were performed in a microfluidic device 5 and microfluidic chip 10 as shown in Figs. 18A to 20. The agent reservoirs of the substrate 1 were preloaded with a combination of linezolid (LIN) and vanomycin (VAN) at a final concentration of 4 $\mu$g/ml and 32 $\mu$g/ml, respectively, NaCl at a final concentration of 340 mM, amoxicillin and clavulanic acid (AMC) at five final concentrations ranging between 32 and 256 $\mu$g/ml, fosfomycin (FOS) at five final concentrations ranging between 32 and 256 $\mu$g/ml, ciprofloxacin (CIP) at five final concentrations ranging between 1 and 128 $\mu$g/ml, nitrofurantoin at five final concentrations ranging between 8 and 128 $\mu$g/ml and trimethoprim (TMP) at five final concentrations ranging between 1 and 64 $\mu$g/ml.

A urine sample was loaded into the microfluidic device 5 and bacterial growth was monitored in the different sets 130 of cell channels. A classification process as shown in Figs. 10 to 13 was performed using the following treatment periods: T1 = 25 min; T2 to T3 = [10, 22] min (average normalized growth rate of all data points in between these two time points were used); T4 = 20 min and T5 = 33 min.

The bacteria in the urine sample were classified as Enterobacterales according to the results of the classification process as shown in Figs. 10 to 13 (Tr1: LIN/-VAL indicated gram negative and Tr2: NaCl indicated gram negative). The clinical breakpoint for AST as published by European Committee on AST for Enterobacteriales and the antibiotic CIP was applied: susceptible if MIC $\leq$ 0.25 $\mu$g/ml, resistant if MIC > 0.5 $\mu$g/ml.

The MIC value of CIP as test antimicrobial agent was determined by generating a dose response curve by mapping the growth rate impact of the different CIP concentrations (1 to 128 $\mu$g/ml), where growth rate impact = 1 - average growth rate of CIP-treated bacterial cells normalized by average growth rate of non-treated bacterial cells. An impact concentration was calculated from the intercept of the dose-response curve with a pre-determined threshold, in this example 0.2. This impact concentration was then mapped to a MIC value using a pre-defined calibration curve, see Fig. 17, to get a MIC value of 0.0625 $\mu$g/ml.

The determined CBP and MIC values were compared and the bacteria in the urine sample were determined to be susceptible to CIP since the determined MIC value of 0.0625 $\mu$g/ml was less than 0.25 $\mu$g/ml.

In parallel, the urine sample was tested with a reference method. Urine (40 $\mu$l) was diluted 1:25 and then 50 $\mu$l was streaked on agar plate, incubated (16-24 h at 37°C) to isolate and count colonies (counted >100, indicating > $5\times10^4$ CFU/mL clinical cutoff applied by the system). This confirmed presence of bacteria in the urine sample. The isolates that grew on the agar were confirmed to be an *Escherichia coli* by matrix-assisted laser desorption/ionization time-of-flight (MALDI-TOF) mass spectrometry (MS) used in the standard of care at Karolinska Microbiology Laboratory (J Microbiol Methods. 2017, 136: 17-20).

Decision was taken that clinical breakpoint for Enterobacterales clinical breakpoint for AST (published by European Committee on AST) to be applied. For antibiotic CIP: susceptible if MIC $\leq$ 0.25 $\mu$g/ml, resistant if MIC > 0.5 $\mu$g/ml.

Broth Micro dilution was performed to determine MIC of the isolate for the antibiotic CIP using commercially available Thermo Fisher Sensititre broth microdilution plates following manufacturer's instruction. The MIC value was determined to be 0.0625 $\mu$g/ml.

The MIC value was below the CBP for CIP (0.0625 $\mu$g/ml < 0.25 $\mu$g/ml) and the isolate was determined to be susceptible to CIP.

The process of the invention gave the same result as the reference method but was able to provide the result after about 30 min instead of 2 days for the reference method. Furthermore, the process of the invention is able to determine MIC values of not only CIP but also of AMC, FOS, TMP and nitrofurantoin in parallel. Hence, MIC values for five different antibiotics are obtained in parallel in addition to the classification of the bacterial cells in only about 30 min.

EXAMPLE 2

This example compared the classification results by the method of the embodiments (Figs. 10 to 13) with a reference method (urine culture and MALDI-TOF MS identification).

*Reference sample 1*

A reference sample comprising *Escherichia coli* ATCC 8739 (gram negative) bacteria at a concentration of $10^5$ CFU/ml was tested by the method of the embodiments (Figs. 10 and 13). The bacteria in the reference sample were classified as gram negative bacteria within 30 min.

*Reference sample 2*

A reference sample comprising *Staphylococcus saprophyticus* CCUG-3706 (gram positive) bacteria at a concentration of $10^5$ CFU/ml was tested by the method of embodiment (Figs. 10 to 13). The bacteria in the reference sample were classified as gram positive within 30 min and as Staphylococcus within 45 min.

*Reference sample 3*

A reference sample comprising *Enterococcus faecalis* CCUG-9997 (gram positive) bacteria at a concentration of $10^5$ CFU/ml was tested by the method of embodiment (Figs. 10 to 13). The bacteria in the reference sample were classified as gram positive within 30 min and as Enterococcus within 45 min.

*Patent sample 1*

A patient sample was tested with the reference method (urine culture and MALDI-TOF MS identification) and the reference method indicated that the patient sample contained *Staphylococcus saprophyticus* (gram positive). The patient sample was also tested by the method of the embodiments (Figs. 10 to 13) and the result was gram positive and *Staphylococcus.*

*Patient sample 2*

A patient sample was tested with a reference method (urine culture and MALDI-TOF MS identification) and the reference method indicated that the patient sample contained *Escherichia coli* (gram negative). The patient sample was also tested by the method of the embodiments (Figs. 10 to 13) and the result was gram negative and Enterobacterales.

*Patient sample 3*

A patient sample was tested with a reference method (urine culture and MALDI-TOF MS identification) and the reference method indicated that the patient sample contained *Enterococcus faecalis* (gram positive). The patient sample was also tested by the method of the embodiments (Figs. 10 to 13) and the result was gram positive and *Enterococcus.*

EXAMPLE 3

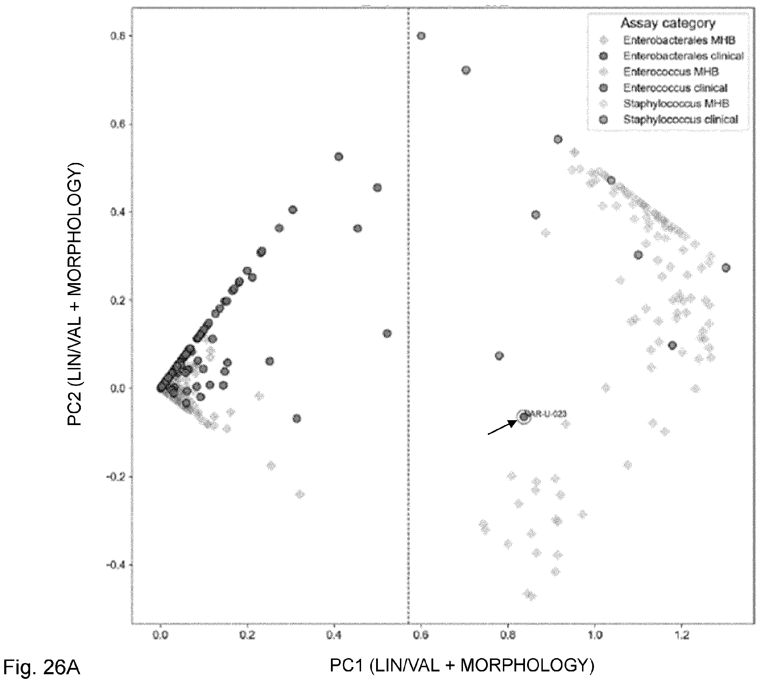

The embodiment of the gram classification as shown in Fig. 5 was tested on both clinical and spiked urine samples (N=676) to investigate the accuracy in gram classification. Fig. 26A shows that out of the 676 sample, a single biological sample indicated by the arrow in the figure was misclassified according to the invention.

In more detail, PC1 and PC2 in Fig. 26A are two vectors used in the calibration to maximize the separation between the two gram types (gram positive versus gram negative). In this case it does not really make sense to call the two vectors principal components, as we only work in two dimensions (gram positive versus gram negative), while principal components usually refer to this type of vectors in three dimensions and higher. PC1 is the actual vector in the LDA part of the gram test (see Fig. 5), and PC2 is only used for visualization purposes. All data points are projected down on the PC1 vector, and the dashed line indicates the threshold value used to separate the two gram types.

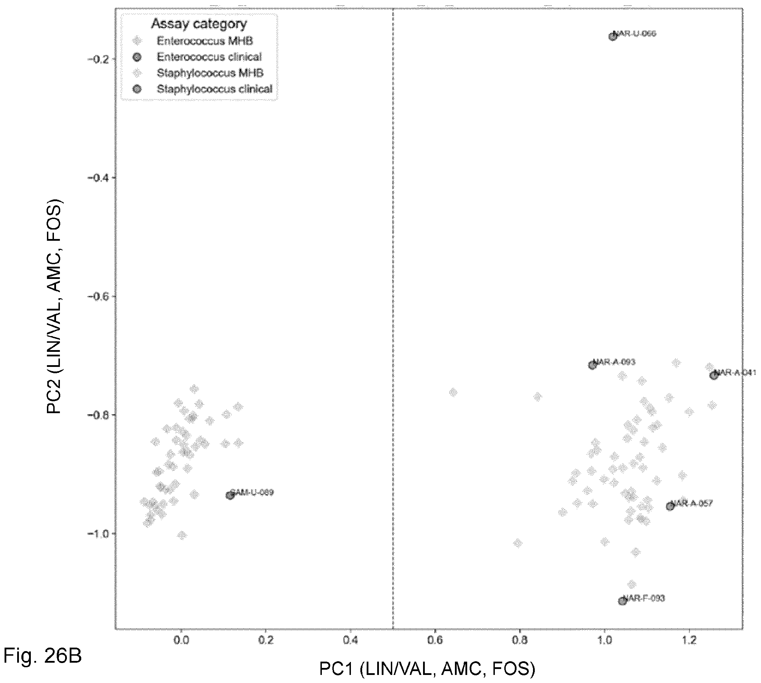

For those biological samples that were classified as gram positive, a genus classification was run as shown in Fig. 6 to classify them as either *Staphylococcus* or *Enterococcus.* For this genus classification, LIN/VAN impact, AMC impact, FOS impact and salt impact were used. The salt impact was determined to be most noisy so that if at least two of the other impact data were available, the salt impact was not included in the genus classification. Fig. 26B illustrates the results of classifying 130 previously classified gram positive biological samples using the LINNAL impact, the AMC impact and the FOS impact.

In more detail, PC1 and PC2 in Fig. 26B are the first two principal components, eigenvectors of the covariance matrix of the data, used in the calibration to maximize the variance of the projected data, and at the same time maximize the separation between the two bacterial types. In this case, PC1 (with the highest variance) is the actual vector used in the LDA part of the gram positive classification part (see lower part of Fig. 6), and PC2 is another vector perpendicular to PC1, only used for visualization purposes. All data points are projected down on the PC1 vector, and the dashed line indicates the threshold value used to separate the two bacterial categories.

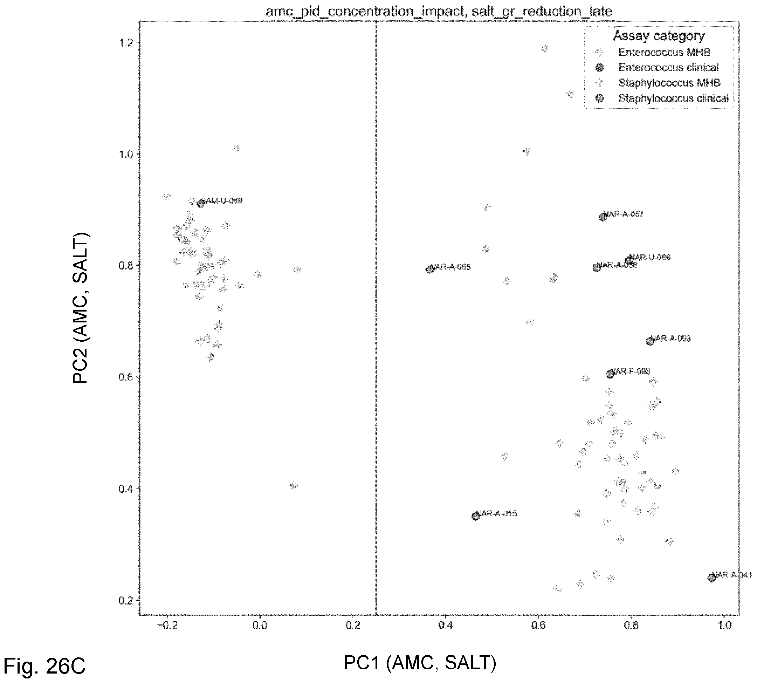

Fig. 26C illustrates the result of classifying the 130 previously classified gram positive biological sample using the AMC impact and the salt growth rate reduction, simulating the case where no valid LINNAL and FOS data are available.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible. The scope of the present invention is, however, defined by the appended claims.

**Claims**

1. A method for classifying bacteria, the method comprising:

   exposing (S1) bacterial cells from a biological sample to a first antimicrobial agent or mixture;
   exposing (S2) bacterial cells from the biological sample to (i) a second antimicrobial agent or mixture and/or (ii) a salt or salt mixture; and
   classifying (S5) the bacterial cells from the biological sample at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture.

2. The method according to claim 1, wherein classifying (S5) the bacterial cells comprises:

classifying (S5) the bacterial cells at least based on the phenotype characteristic response of the bacterial cells to the first antimicrobial agent or mixture and the phenotype characteristic response of the bacterial cells to the second antimicrobial agent or mixture if valid data of the phenotype characteristic response of the bacterial cells to the second antimicrobial agent or mixture is available; and
classifying (S5) the bacterial cells at least based on the phenotype characteristic response of the bacterial cells to the first antimicrobial agent or mixture and the phenotype characteristic response of the bacterial cells to the salt or salt mixture if no valid data of the phenotype characteristic response of the bacterial cells to the second antimicrobial agent or mixture is available.

3. The method according to claim 1 or 2, wherein classifying (S5) the bacterial cells comprises classifying (S12) the bacterial cells, optionally as *Staphylococcus* or *Enterococcus,* at least based on (i) the phenotype characteristic response of the bacterial cells to the first antimicrobial agent or mixture, and (iia) the phenotype characteristic response of the bacterial cells to the second antimicrobial agent or mixture and/or (iib) the phenotype characteristic response of the bacterial cells to the salt or salt mixture if the bacterial cells have a predefined gram classification, preferably gram positive.

4. The method according to claim 3, further comprising:

exposing (S3) bacterial cells from the biological sample to a third antimicrobial agent or mixture, wherein
classifying (S12) the bacterial cells comprises classifying (S12) the bacterial cells based on at least two of the phenotype characteristic response of the bacterial cells to the first antimicrobial agent or mixture, the phenotype characteristic response of the bacterial cells to the second antimicrobial agent or mixture, the phenotype characteristic response of the bacterial cells to the salt or salt mixture, and a phenotype characteristic response of the bacterial cells to the third antimicrobial agent or mixture.

5. The method according to claim 4, wherein classifying (S12) the bacterial cells comprises:

classifying (S12) the bacterial cells based on the phenotype characteristic responses of the bacterial cells to at least two of the first, second and third antimicrobial agents or mixtures if valid data of the phenotype characteristic responses of the bacterial cells to the at least two of the first, second and third antimicrobial agents or mixtures are available; and
classifying (S12) the bacterial cells based on the phenotype characteristic response of the bacterial cells to the salt or salt mixture and the phenotype characteristic response of the bacterial cells to one of the first, second and third antimicrobial agents or mixtures if valid data of the phenotype characteristic response of the bacterial cells to merely one of the first, second and third antimicrobial agents or mixtures is available.

6. The method according to any one of claims 1 to 5, wherein classifying (S5) the bacterial cells comprises:

determining (S10) a gram classification for the bacterial cells at least based on the phenotype characteristic response of the bacterial cells to an antimicrobial agent or mixture, preferably the first antimicrobial agent or mixture, at least one morphology characteristic of the bacterial cells from the biological sample, or the phenotype characteristic response of the bacterial cells to the salt or salt mixture; and
optionally classifying (S50) the bacterial cells as Enterobacterales if the determined gram classification is gram negative.

7. The method according to claim 6, wherein determining (S10) the gram classification comprises determining (S22) the gram classification for the bacterial cells at least based on the phenotype characteristic response of the bacterial cells to the antimicrobial agent or mixture and the at least one morphology characteristic of the bacterial cells from the biological sample if valid data of the at least one morphology characteristic of the bacterial cells and valid data of the phenotype characteristic response of the bacterial cells to the antimicrobial agent or mixture are available.

8. The method according to claim 6 or 7, wherein determining (S10) the gram classification comprises determining (S23) the gram classification for the bacterial cells at least based on the at least one morphology characteristic of the bacterial cells from the biological sample if valid data of the at least one morphology characteristic of the bacterial cells is available but no valid data of the phenotype characteristic response of the bacterial cells to the antimicrobial agent or mixture is available.

9. The method according to any one of claims 6 to 8, wherein determining (S10) the gram classification comprises determining (S25) the gram classification for the bacterial cells at least based on the phenotype characteristic response of the bacterial cells to the antimicrobial agent or mixture if valid data of the phenotype characteristic response of the bacterial cells to the antimicrobial agent or mixture is available but no valid data of the at least one morphology characteristic of the bacterial cells is available.

10. The method according to any one of claims 6 to 9, wherein determining (S10) the gram classification comprises determining (S10) the gram classification for the bacterial cells at least based on the phenotype characteristic response of the bacterial cells to the salt or salt mixture if no valid data of the at least one morphology characteristic of the bacterial cells and no valid data of the phenotype characteristic response of the bacterial cells to the antimicrobial agent or mixture are available.

11. A method for analyzing bacteria, the method comprises:

exposing (S60) bacterial cells from a biological sample to a test antimicrobial agent;
classifying (S61) the bacterial cells according to any one of claims 1 to 10;
determining (S62) a clinical breakpoint for the test antimicrobial agent based on a result of classifying the bacterial cells;
determining (S63) a minimum inhibitory concentration of the test antimicrobial agent based on a phenotype characteristic response of the bacterial cells to the test antimicrobial agent;
comparing (S64) the minimum inhibitory concentration with the clinical breakpoint; and
optionally determining (S65) whether the bacterial cells are susceptible to the test antimicrobial agent based on the comparison between the minimum inhibitory concentration and the clinical breakpoint.

12. The method of claim 11, wherein

exposing (S60) the bacterial cells to the test antimicrobial agent comprises exposing (S60) bacterial cells captured in a test set or sets (130) of cell channels (140) having a respective channel restriction (145) configured to restrict bacterial cells entering the cell channels (140) from leaving the cell channels (140) to the test antimicrobial agent;
exposing (S1) the bacterial cells to the first antimicrobial agent or mixture comprises exposing (S1) bacterial cells captured in a first set or sets (130) of cell channels (140) having a respective channel restriction (145) configured to restrict bacterial cells entering the cell channels (140) from leaving the cell channels (140) to the first antimicrobial agent or mixture; and
exposing (S2) the bacterial cells to the second antimicrobial agent or mixture comprises exposing (S2) bacterial cells captured in a second set or sets (130) of cell channels (140) having a respective channel restriction (145) configured to restrict bacterial cells entering the cell channels (140) from leaving the cell channels (140) to the second antimicrobial agent or mixture, and
exposing (S2) the bacterial cells to the salt or salt mixture comprises exposing (S2) bacterial cells captured in a third set or sets (130) of cell channels (140) having a respective channel restriction (145) configured to restrict bacterial cells entering the cell channels (140) from leaving the cell channels (140) to the salt or salt mixture; and
optionally wherein the test set or sets (130) of cell channels (140), the first set or sets (130) of cell channels (140), the second set or sets (130) of cell channels (140) and the third set or sets (130) of cell channels (140) are formed as separate compartments in a microfluidic chip (10).

13. The method according to any one of the claims 1 to 12, wherein the phenotype characteristic response(s) is selected from the group consisting of growth rate, degree of nucleoid compaction, degree of metabolic activity and degree of membrane integrity.

14. An analyzer (200) comprising:

a pressure source (260) configured to apply pressure to a microfluidic device (5),
a processor (220); and
a memory (230) comprising instructions executable by the processor (220) to cause the processor (230) to:

control the pressure source (260) to expose bacterial cells from a biological sample to a first antimicrobial agent or mixture in the microfluidic device (5);
control the pressure source (260) to expose bacterial cells from the biological sample to (i) a second antimicrobial agent or mixture and/or (ii) a salt or salt mixture in the microfluidic device (5); and
classify the bacterial cells from the biological sample at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture.

15. An analyzer (200) comprising:

a pressure source (260) configured to apply pressure to a microfluidic device (5),
a processor (220); and
a memory (230) comprising instructions executable by the processor (220) to cause the processor (230) to:

control the pressure source (260) to expose bacterial cells from a biological sample to a test antimicrobial agent in the microfluidic device (5);
control the pressure source (260) to expose bacterial cells from the biological sample to a first antimicrobial agent or mixture in the microfluidic device (5);
control the pressure source (260) to expose bacterial cells from the biological sample to (i) a second antimicrobial agent or mixture and/or (ii) a salt or salt mixture in the microfluidic device (5);
classify the bacterial cells at least based on (i) a phenotype characteristic response of the bacterial cells to the first antimicrobial agent or mixture, and (iia) a phenotype characteristic response of the bacterial cells to the second antimicrobial agent or mixture and/or (iib) a phenotype characteristic response of the bacterial cells to the salt or salt mixture;
determine a clinical breakpoint for the test antimicrobial agent based on a result of classifying the bacterial cells;
determine a minimum inhibitory concentration of the test antimicrobial agent based on a phenotype characteristic response of the bacterial cells to the test antimicrobial agent; and
compare the minimum inhibitory concentration with the clinical breakpoint.

Fig. 1

EP 4 481 054 A1

```
              ┌─────────────┐
              │    START    │
              └──────┬──────┘
                     │
                     ▼
         ┌───────────────────────────────┐
    S1   │ EXPOSE BACTERIAL CELLS TO 1ST  │
         │ ANTIMICROBIAL AGENT/MIXTURE    │
         └───────────────┬───────────────┘
                         │
                         ▼
         ┌───────────────────────────────┐
    S2   │ EXPOSE BACTERIAL CELLS TO 2ND  │
         │ ANTIMICROBIAL AGENT/MIXTURE    │
         │ AND/OR TO SALT/SALT MIXTURE    │
         └───────────────┬───────────────┘
                         │
                         ▼
         ┌ ─ ─ ─ ─ ─ ─ ─ ─ ─ ─ ─ ─ ─ ─ ┐
    S3   │ EXPOSE BACTERIAL CELLS TO 3RD  │
         │ ANTIMICROBIAL AGENT/MIXTURE    │
         └ ─ ─ ─ ─ ─ ─ ─ ┬ ─ ─ ─ ─ ─ ─ ┘
                         │
                         ▼
         ┌ ─ ─ ─ ─ ─ ─ ─ ─ ─ ─ ─ ─ ─ ─ ┐
    S4   │   DETERMINE MORPHOLOGY         │
         │   CHARACTERISTIC              │
         └ ─ ─ ─ ─ ─ ─ ─ ┬ ─ ─ ─ ─ ─ ─ ┘
                         │
                         ▼
         ┌───────────────────────────────┐
    S5   │  CLASSIFY BACTERIAL CELLS      │
         └───────────────┬───────────────┘
                         │
                         ▼
                  ┌─────────────┐
                  │    STOP     │
                  └─────────────┘
```

Fig. 2

```
         ┌───────────────────────────────┐
   S10   │    DETERMINE GRAM              │
         │    CLASSIFICATION             │
         └───────────────┬───────────────┘
                         │
                         ▼
         ┌ ─ ─ ─ ─ ─ ─ ─ ─ ─ ─ ─ ─ ─ ─ ┐
   S11   │    DETERMINE GENUS            │
         │    CLASSIFICATION            │
         └ ─ ─ ─ ─ ─ ─ ─ ┬ ─ ─ ─ ─ ─ ─ ┘
                         │
                         ▼
         ┌───────────────────────────────┐
   S12   │    CLASSIFY BACTERIAL          │
         │    CELLS                      │
         └───────────────────────────────┘
```

Fig. 3

Fig. 4

EP 4 481 054 A1

Fig. 5

Fig. 6

```
S30  ┌─────────────────────┐
     │   DETERMINE 1ST     │
     │  CANDIDATE GRAM     │
     │  CLASSIFICATION     │
     └─────────────────────┘
              │
              ▼
S31  ┌─────────────────────┐
     │   DETERMINE 2ND     │
     │  CANDIDATE GRAM     │
     │  CLASSIFICATION     │
     └─────────────────────┘
              │
              ▼
S32  ┌─────────────────────┐
     │  DETERMINE GRAM     │
     │   CLASIFICATION     │
     └─────────────────────┘
```

Fig. 7

```
S40  ┌─────────────────────┐
     │   DETERMINE 1ST     │
     │ CANDIDATE GENUS     │
     │  CLASSIFICATION     │
     └─────────────────────┘
              │
              ▼
S41  ┌─────────────────────┐
     │   DETERMINE 2ND     │
     │ CANDIDATE GENUS     │
     │  CLASSIFICATION     │
     └─────────────────────┘
              │
              ▼
S42  ┌─────────────────────┐
     │  DETERMINE GENUS    │
     │   CLASIFICATION     │
     └─────────────────────┘
```

Fig. 8

EP 4 481 054 A1

S50 CLASSIFY BACTERIAL
CELLS AS 1ST ORDER

S52 CLASSIFY BACTERIAL
CELLS AS 2ND GENUS

S51 CLASSIFY BACTERIAL
CELLS AS 1ST GENUS

Fig. 9

Fig. 10

Fig. 11

GRAM TEST
RESULTS FROM Tr1
=
GRAM TEST
RESULTS FROM Tr2

YES

SELECT GRAM
TEST RESULT
FROM Tr1/Tr2

NO

SELECT GRAM
NEGATIVE

GENUS TEST RESULTS
FROM Tr3
=
GENUS TEST RESULTS
FROM Tr4

YES

SELECT GENUS TEST
RESULT FROM Tr3/Tr4

NO

USE NaCl BASED
GENUS
CLASSIFICATION

Tr2: NaCl

Tr2 IMPACT AT T2
(TO T3)
>
GENUS CUTOFF 3

YES

SELECT
*ENTEROCOCCUS*

NO

SELECT
*STAPHYLOCOCCUS*

Fig. 12

```
                    ┌──────────────┐                              ┌──────────────────────┐
                    │   GRAM       │                              │   pID =              │
              ┌─────│   NEGATIVE   │──────────────────────────────│   ENTEROBACTERALES   │
              │     └──────────────┘                              └──────────────────────┘
 ┌──────────┐ │
 │ GRAM TEST│ │
 │ RESULT   │─┤                                                           ┌──────────────────────┐
 └──────────┘ │                                                           │   pID =              │
              │                                                      ┌────│   STAPHYLOCOCCUS     │
              │     ┌──────────────┐     ┌──────────────────┐        │    └──────────────────────┘
              └─────│   GRAM       │─────│   GENUS TEST     │────────┤
                    │   POSITIVE   │     │   RESULT         │        │    ┌──────────────────────┐
                    └──────────────┘     └──────────────────┘        │    │   pID =              │
                                                                     └────│   ENTEROCOCCUS       │
                                                                          └──────────────────────┘
```

Fig. 13

EP 4 481 054 A1

START

S60  EXPOSE BACTERIAL CELLS
     TO TEST ANTIMICROBIAL
     AGENT

S61  STEPS S1-S5

S62  DETERMINE CBP

S63  DETERMINE MIC

S64  COMPARE MIC AND CBP

STOP

Fig. 14

FROM STEP S64

S65  DETERMINE
     SUSCEPTIBILITY

STOP

Fig. 15

Dose-response curves - impact concentration

Fig. 16

Fig. 17

Fig. 18B

Fig. 18A

Fig. 18C

EP 4 481 054 A1

Fig. 19

Fig. 20

Fig. 21

Fig. 23

Fig. 22

EP 4 481 054 A1

210

5

200

Fig. 24

Fig. 25

Fig. 26A

Fig. 26B

Fig. 26C

Europäisches
Patentamt

European
Patent Office

Office européen
des brevets

# EUROPEAN SEARCH REPORT

**Application Number**

EP 24 17 6483

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/298408 A1 (ROLAIN JEAN-MARC [FR] ET AL) 19 October 2017 (2017-10-19) * paragraph [0016] - paragraph [0051] * * paragraphs [0056], [0077], [0082]; claims 1,10; figures 1A,1B; tables C1,C2 * | 1,3-5,11 | INV. C12Q1/04 |
| X | WO 2022/025864 A1 (HEWLETT PACKARD DEVELOPMENT CO [US]) 3 February 2022 (2022-02-03) | 1-13 | |
| Y | * paragraphs [0006], [0017], [0028], [0077]; claims 1,30,31 * | 1-15 | |
| X | WO 2016/137341 A1 (MASTAPLEX LTD [NZ]) 1 September 2016 (2016-09-01) * claims 16,17 * | 1 | |
| X | Aryal Sagar: "Classification of Bacteria in different 9 ways", , 6 June 2023 (2023-06-06), pages 1-19, XP093211541, Retrieved from the Internet: URL:https://microbenotes.com/classification-of-bacteria/ * pages 1-4,8,9 * | 1-10 | |
| X,D | US 10 041 104 B2 (ELF JOHAN [SE]; ASTREGO DIAGNOSTICS AB [SE]) 7 August 2018 (2018-08-07) * claims 11,12 * | 1 | **TECHNICAL FIELDS SEARCHED (IPC)** C12Q |
| Y | WO 2016/063199 A1 (ECOLE POLYTECH [CH]) 28 April 2016 (2016-04-28) * abstract; claim 1 * * paragraph [0068] * | 1-15 | |

The present search report has been drawn up for all claims

| Place of search | Date of completion of the search | Examiner |
|---|---|---|
| The Hague | 3 October 2024 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
....................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 6483

This annex lists the patent family members relating to the patent documents cited in the above-mentioned European search report.
The members are as contained in the European Patent Office EDP file on
The European Patent Office is in no way liable for these particulars which are merely given for the purpose of information.

03-10-2024

| Patent document cited in search report | | | Publication date | Patent family member(s) | | | Publication date |
|---|---|---|---|---|---|---|---|
| US 2017298408 | A1 | | 19-10-2017 | CN | 107002115 | A | 01-08-2017 |
| | | | | EP | 3204508 | A1 | 16-08-2017 |
| | | | | FR | 3026745 | A1 | 08-04-2016 |
| | | | | JP | 6626508 | B2 | 25-12-2019 |
| | | | | JP | 2017531449 | A | 26-10-2017 |
| | | | | US | 2017298408 | A1 | 19-10-2017 |
| | | | | WO | 2016055724 | A1 | 14-04-2016 |
| WO 2022025864 | A1 | | 03-02-2022 | TW | 202219276 | A | 16-05-2022 |
| | | | | WO | 2022025864 | A1 | 03-02-2022 |
| WO 2016137341 | A1 | | 01-09-2016 | AU | 2016224107 | A1 | 12-10-2017 |
| | | | | BR | 112017018259 | A2 | 10-04-2018 |
| | | | | CA | 2977478 | A1 | 01-09-2016 |
| | | | | CL | 2017002176 | A1 | 13-07-2018 |
| | | | | CN | 107532194 | A | 02-01-2018 |
| | | | | EP | 3262183 | A1 | 03-01-2018 |
| | | | | ES | 2952442 | T3 | 31-10-2023 |
| | | | | JP | 6940417 | B2 | 29-09-2021 |
| | | | | JP | 2018507712 | A | 22-03-2018 |
| | | | | PL | 3262183 | T3 | 04-12-2023 |
| | | | | US | 2018245124 | A1 | 30-08-2018 |
| | | | | US | 2021172001 | A1 | 10-06-2021 |
| | | | | WO | 2016137341 | A1 | 01-09-2016 |
| | | | | ZA | 201706277 | B | 31-05-2023 |
| US 10041104 | B2 | | 07-08-2018 | AU | 2015288329 | A1 | 12-01-2017 |
| | | | | AU | 2015288389 | A1 | 12-01-2017 |
| | | | | BR | 112017000167 | A2 | 16-01-2018 |
| | | | | BR | 112017000168 | A2 | 16-01-2018 |
| | | | | CA | 2954360 | A1 | 14-01-2016 |
| | | | | CA | 2954378 | A1 | 14-01-2016 |
| | | | | CN | 106471122 | A | 01-03-2017 |
| | | | | CN | 106661530 | A | 10-05-2017 |
| | | | | DK | 3167044 | T3 | 02-12-2019 |
| | | | | EP | 3167044 | A1 | 17-05-2017 |
| | | | | EP | 3167061 | A1 | 17-05-2017 |
| | | | | ES | 2762914 | T3 | 26-05-2020 |
| | | | | JP | 6600349 | B2 | 30-10-2019 |
| | | | | JP | 6629287 | B2 | 15-01-2020 |
| | | | | JP | 7066206 | B2 | 13-05-2022 |
| | | | | JP | 2017519517 | A | 20-07-2017 |
| | | | | JP | 2017520262 | A | 27-07-2017 |
| | | | | JP | 2020054365 | A | 09-04-2020 |
| | | | | PL | 3167044 | T3 | 24-08-2020 |
| | | | | US | 2017137861 | A1 | 18-05-2017 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 24 17 6483

This annex lists the patent family members relating to the patent documents cited in the above-mentioned European search report.
The members are as contained in the European Patent Office EDP file on
The European Patent Office is in no way liable for these particulars which are merely given for the purpose of information.

03-10-2024

| Patent document cited in search report | Publication date | Patent family member(s) | | Publication date |
|---|---|---|---|---|
| | | US | 2017159048 A1 | 08-06-2017 |
| | | US | 2018320216 A1 | 08-11-2018 |
| | | WO | 2016007063 A1 | 14-01-2016 |
| | | WO | 2016007068 A1 | 14-01-2016 |
| WO 2016063199 A1 | 28-04-2016 | DK | 3505638 T3 | 15-02-2021 |
| | | EP | 3209790 A1 | 30-08-2017 |
| | | EP | 3505638 A1 | 03-07-2019 |
| | | ES | 2795991 T3 | 25-11-2020 |
| | | ES | 2850881 T3 | 01-09-2021 |
| | | US | 2017312748 A1 | 02-11-2017 |
| | | US | 2023149927 A1 | 18-05-2023 |
| | | WO | 2016063199 A1 | 28-04-2016 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10041104 B
- US 10570437 B
- US 10913969 B
- US 8828680 B

**Non-patent literature cited in the description**

- **WANG et al.** *Current Biology*, 2010, vol. 20, 1099-1103
- **BALTEKIN et al.** *PNAS*, 2017, vol. 114 (34), 9170-9175
- *J Microbiol Methods.*, 2017, vol. 136, 17-20